# EUROPEAN PATENT SPECIFICATION

(11) **EP 2 812 690 B1**
(45) Date of publication and mention of the grant of the patent: **26.07.2017**
(21) Application number: 13704627.2
(22) Date of filing: 07.02.2013
(51) Int. Cl.: G01N 33/50

(54) **DIAGNOSING MULTIPLE SCLEROSIS**
DIAGNOSE MULTIPLER SKLEROSE
DIAGNOSTIC DE LA SCLÉROSE EN PLAQUES

(30) Priority: 07.02.2012 GB 201202092
(43) Date of publication of application: 17.12.2014
(73) Proprietor: Oxford University Innovation Limited, Botley Oxford OX2 0JB (GB)
(72) Inventor: ANTHONY, Daniel, Oxford Oxfordshire OX1 3QT (GB); SIBSON, Nicola, Oxford Oxfordshire OX3 7DQ (GB); PALACE, Jacqueline, Oxford Oxfordshire OX3 9DU (GB)
(74) Representative: MacLean, Martin Robert
(86) International application number: PCT/GB2013/050285
(87) International publication number: WO 2013/117930

(56) References cited:
- WO-A1-2011/067243
- NICOLI F ET AL: "Cerebrospinal fluid metabolic profiles in multiple sclerosis and degenerative dementias obtained by high resolution proton magnetic resonance spectroscopy", COMPTES RENDUS DES SEANCES DE L'ACADEMIE DES SCIENCES.SERIE III: SCIENCES DE LA VIE, ELSEVIER, AMSTERDAM, NL, vol. 319, no. 7, 1 June 1996 (1996-06-01), pages 623-631, XP008091327, ISSN: 0764-4469
- ALEXANDRA J. SINCLAIR ET AL: "NMR-based metabolomic analysis of cerebrospinal fluid and serum in neurological diseases - a diagnostic tool?", NMR IN BIOMEDICINE, 18 August 2009 (2009-08-18), pages n/a-n/a, XP055058316, ISSN: 0952-3480, DOI: 10.1002/nbm.1428
- NORBERT W. LUTZ ET AL: "Inflammatory Multiple-Sclerosis Plaques Generate Characteristic Metabolic Profiles in Cerebrospinal Fluid", PLOS ONE, vol. 2, no. 7, 4 July 2007 (2007-07-04), page e595, XP055058328, DOI: 10.1371/journal.pone.0000595
- BAS JASPERSE ET AL: "N-acetylaspartic acid in cerebrospinal fluid of multiple sclerosis patients determined by gas-chromatography-mass spectrometry", JOURNAL OF NEUROLOGY, STEINKOPFF-VERLAG, DA, vol. 254, no. 5, 6 April 2007 (2007-04-06) , pages 631-637, XP019517014, ISSN: 1432-1459, DOI: 10.1007/S00415-006-0415-5

## Description

The present invention relates to methods of diagnosing disorders of the central nervous system (CNS) using metabolite profiling. In particular, the present invention relates to methods of diagnosing Multiple Sclerosis (MS) in human subjects.

Multiple sclerosis (MS) is the commonest cause of progressive disability in the Western world. From onset of the condition, the clinical phenotype of MS follows either a relapsing remitting (RR MS) course, or a progressive course (primary progressive (PP) MS). After a period of time in the RR phase the majority of patients develop a secondary progressive (SP) phase. It is during the progressive phases that the majority of disability accrues. It appears that inflammation predominates during the relapsing phase, whereas neurodegeneration is the pathological substrate of disability during the progressive phase. However, there is much overlap.

Unfortunately, current disease modifying agents targeting inflammatory mechanisms are not effective in progressive disease, and new treatment strategies are required. Exploring differences between the MS phenotypes may identify key mechanisms driving progression, and is the first step in developing effective new therapies to prevent disability.

The currently used main outcome measures in MS consist of clinical scales and magnetic resonance imaging (MRI). These are associated with a number of disadvantages. The clinical outcomes have low sensitivity and poor pathogenic specificity, and are open to subjective distortion due to patients and clinician subjectivity. A neuroprotective trial measuring clinical disability outcome may require 600 or more patients over two or even three years. MRI has been used to study inflammatory lesions and in studies of antiinflammatory therapies. Atrophy is a surrogate marker for neuroprotection; however, it lacks sensitivity and is difficult to measure in patients with severe or advanced disease when the atrophy is marked. Additionally, pseudoatrophy is seen when inflammation is reduced such as with immunomodulatory drugs. Changes in myelin and damage in normal-appearing brain tissue are recognised pathologically, but are not easy to measure due to difficulty in measuring whole brain and spinal cord load.

Identifying specific biomarkers in biological fluids has been considered across a number of diseases, both in animal models and humans. Urines from patients with neurological disease, including MS, have been assayed for disease-specific markers, such as myelin basic protein-derived material indicating demyelination (Whitaker et al., 1994; Whitaker et al., 1995). However, these studies focussed on the detection of a single specific metabolite, and although differences have been identified at a group level, predictive value at an individual level has been low.

WO 2011/067243 A1 describes a method for diagnosing multiple sclerosis in a subject.

There is therefore a need for an improved method of diagnosing MS that has an increased predictive value at an individual level. There is also a need to identify surrogate outcome measures of inflammation and neurodegeneration that may be used to power clinical studies of MS treatments. It would further be advantageous if the improved method could differentiate between different phases (also referred to as stages) of MS.

The present invention addresses one or more of the above problems by providing methods for diagnosing MS in humans.

In one aspect, the invention provides an *in vitro* method for diagnosing MS in a human test subject, comprising:
a.
   (i) determining the relative concentrations of three or more metabolites in a sample from said subject, wherein said three or more metabolites are selected from: blood metabolites, wherein said blood metabolites comprise: phosphocholine, lactate, N-acetyl species, fatty acid, glucose, and L-glutamine;
   (ii) comparing the relative concentrations of said three or more metabolites in the sample with the relative concentrations of the same metabolites in at least one reference standard from a subject that does not have MS;
      and
   (iii) identifying a concentration difference for each of said three or more metabolites in the sample relative to the reference standard, wherein said concentration difference is selected from: an increase in the concentration of one or more blood metabolites selected from: fatty acid, N-acetyl species, and lactate; and
      a decrease in the concentration of the blood metabolites glucose and phosphocholine; or
      wherein said concentration difference is selected from: an increase in the concentration of one or more blood metabolites selected from: fatty acid, N-acetyl species, and L-glutamine;
      and
      a decrease in the concentration of the blood metabolites glucose, and phosphocholine;
      wherein said concentration differences correlate with the presence of MS;
   wherein said N-acetyl species is N-acetyl aspartate and/or N-acetyl glycoprotein; and/or
   wherein the fatty acid has a resonance value of δ0.90, br, C*H₃*, δ1.30, br, C*H₂*C*H₂*C*H₃*,δ1.55, br, C*H*₂C*H₃*, δ 2.05, br, C*H₂*C*H*=, δ 2.25, br, C*H₂*C*OOH,* δ5.35, br, C*H₂*C*H*= relative to a methyl resonance of 3-trimethylsilyl-1-propionate (TSP) defined at 0.00 ppm as determinable by ¹H-NMR;
   and/or
b.
   (i) determining the relative concentrations of two or more metabolites in a sample from said subject, wherein said two or more metabolites are selected from: urine metabolites, wherein said urine metabolites comprise: citrate, creatinine, lactate and trimethylamine N-oxide (TMAO);
   (ii) comparing the relative concentrations of said two or more metabolites in the sample with the relative concentrations of the same metabolites in at least one reference standard from a subject that does not have MS;
      and
   (iii) identifying a concentration difference for each of said two or more metabolites in the sample relative to the reference standard, wherein said concentration difference is selected from: an increase in the concentration of one or more urine metabolites selected from: TMAO and citrate; and
   a decrease in the concentration of one or more urine metabolites selected from: creatinine, and lactate;
   wherein said concentration differences correlate with the presence of MS.

Using a process of high resolution ¹H-NMR (Nuclear Magnetic Resonance) spectroscopy, coupled with partial least squares discriminate analysis (PLS-DA), the present inventors have identified metabolites that can be used to diagnose MS. Further described herein is differentiation between different specific disease phases of MS. The metabolites are small molecules that are intermediates and/or products of mammalian metabolism.

Two or more (for example, two, three, or four) metabolites selected from: blood metabolites, wherein said blood metabolites comprise: phosphocholine, lactate, N-acetyl species, and beta-hydroxybutyrate; and/or urine metabolites, wherein said urine metabolites comprise: TMAO, citrate, creatinine, and lactate are described herein.

Two or more metabolites (for example, two, three, four, five, or six) selected from: blood metabolites, wherein said blood metabolites comprise: phosphocholine, lactate, N-acetyl species, beta-hydroxybutyrate, fatty acid (preferably fatty acid δ_{x-y}0.88, 1.30, 5.35), and glucose; and/or urine metabolites, wherein said urine metabolites comprise: TMAO, citrate, creatinine, and lactate are described herein.

Two or more metabolites selected from: blood metabolites, wherein said blood metabolites comprise: L-glutamine, alanine, glucose, phosphocholine,
fatty acid, N-acetyl species, lactate, and beta-hydroxybutyrate; and/or urine metabolites, wherein said urine metabolites comprise: TMAO, citrate, creatinine, and lactate are described herein.

In one embodiment, "N-acetyl species" means at least one of the following metabolites: N-acetyl aspartate, and N-acetyl glycoprotein.

In one embodiment, "glucose" comprises alpha-glucose and beta-glucose. In one embodiment, glucose is alpha-glucose. In one embodiment, glucose is beta-glucose.

In one embodiment, said blood metabolites further comprise a singlet (δ_{x-y}3.37) and a singlet (δ_{x-y}2.65) as measured using ¹H-NMR.

In one embodiment, said metabolites are associated with the following parameters, as measured using ¹H-NMR (Key: δ - chemical shift, M - multiplicity, A - Assignment of proton, s - singlet, d - doublet, t - triplet, q - quartet, dd - double doublet, ddd - double double doublet, m - multiplet, br - broad resonance, δ_{x-y} centre of the bin identified by SIMCA. Resonance values (chemical shift) are quoted relative to the methyl resonance of 3-trimethylsilyl-1-propionate (TSP) defined at 0.00 ppm, and are quoted with an error margin of ± 0.01 ppm):
**alanine** (δ1.46, d, C*H*₃; δ3.73, q, C*H*), **fatty acid** (δ0.90, br, C*H*₃, δ1.30 br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), **α-glucose** (δ3.42, dd, C(4)*H*, δ3.44, dd, C(6)*H*_{A}H_{B}, δ3.54, dd, C(2)*H*, δ3.73, dd, C(3)*H,* δ3.79, dd, C(6)H₄*H*_{B}, δ3.86, m, C(5)*H*, δ5.24, d, C(1)*H*), **β-glucose** (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), **lactate** (δ1.32 d, C*H*₃, δ4.11, q, C*H*), **N-acetyl species** (δ2.03, br s, COC*H*₃), **β-hydroxybutyrate** (δ1.19 d, C*H*₃, δ2.28, m, C*H*_{A}H_{B}, δ2.37, m, CH_{A}*H*_{B}, δ4.12, m, C*H*), **phosphocholine** (δ3.25, s, *N*(C*H*₃)₃, δ3.59, t, NC*H*₂, δ4.17, m, NCH₂C*H*₂), **L-glutamine** (δ2.13, m, C*H*₂CHNH₂, δ2.45, m, OCC*H*₂, δ3.77, t, CH₂C*H*NH₂), **unidentified singlet** (δ_{x-y}3.37), **unidentified singlet** (δ_{x-y}2.65), **citrate** (δ2.50, d, C*H*_{A}H_{B}, δ2.65, d, CH_{A}*H*_{B}), **creatinine** (δ3.05, s, NC*H*₃, δ4.10, s, C*H*₂), and **trimethylamine N-oxide (TMAO)** (δ3.26, s, ON(C*H*₃)₃).

The present inventors have discovered that differences in the concentrations of the above metabolites, as compared to a specific reference standard, are present in blood and/or urine of subjects having MS and that determining the concentrations of said metabolites in blood/urine from a test subject can be used to diagnose MS. Thus, the method of the present invention can be used to diagnose MS. A method permitting diagnosis of a specific disease phase of MS is described herein.

As used herein in relation to MS, "disease phase" refers to the specific MS clinical phenotypes of relapsing remitting (RR) phase MS, primary progressive (PP) phase MS, and secondary progressive (SP) phase MS. Said clinical phenotypes describe the disease course from onset.

The sample that is to be tested using the method of the invention can be derived from blood or urine that has been obtained from a human test subject.

The term blood comprises whole blood, blood serum (henceforth "serum") and blood plasma (henceforth "plasma"). Serum and plasma are derived from blood and thus may be considered as specific subtypes within the broader genus "blood". Processes for obtaining serum or plasma from blood are known in the art. For example, it is known in the art that blood can be subjected to centrifugation in order to separate red blood cells, white blood cells, and plasma. Serum is defined as plasma that lacks clotting factors. Serum can be obtained by centrifugation of blood in which the clotting process has been triggered. Optionally, this can be carried out in specialised centrifuge tubes designed for this purpose.

A sample for use in the method of the present invention can be derived from blood or urine that has undergone processing after being obtained from a test subject. Alternatively, a sample can be derived from blood or urine that has not undergone any processing after being obtained from a test subject.

The method of the present invention thus encompasses the use of samples that have undergone minimal or zero processing before testing. This provides a significant advantage over prior art methods in terms of time, cost and practicality. By way of example, a urine sample obtained from a test subject may be tested directly using the method of the present invention, without further processing. Serum and plasma samples can be readily obtained from blood samples using simple and readily available techniques that are well known in the art, as described above.

The method of the present invention is an *in vitro* diagnostic method. Thus, the method of the present invention can be carried out *in vitro* on an isolated sample that has been obtained from a test subject.

A number of advantages are described herein. The use of samples derived from tissue fluids that are readily accessible allows for samples to be measured longitudinally. Furthermore, methods described herein can advantageously be used to study groups of patients with pure neuroprotective conditions, pure inflammatory conditions and different phases and types of MS, thus permitting the separation out of pathological processes for measurement. For example, a useful outcome would be "a move from the relapsing remitting phase of MS to the secondary progressive phase" (the former early stage causes little permanent disability whereas the latter is where the disability is progressively accrued). At present this transfer is gradual and not clinically clear, such that a date can only be retrospectively identified and only within 12-18 months. Thus, by analysing the biomarkers of these two phases using methods described herein, it is possible to use a time to transfer as an outcome. Additionally, by using methods described herein to separate out early versus late MS, it is possible to identify differentiating markers that can measure disease progression. Similarly, the method of the invention can be used to identify diagnostic specific features relevant to MS versus other inflammatory conditions and the same with neurodegenerative conditions. This provides the identification of relevant aetiological markers that can be used diagnostically, and of treatment targets. New biomarkers for evaluating neuroprotective treatments are described herein.

A blood metabolite is a metabolite that can be identified in blood (including serum and plasma). A urine metabolite is a metabolite that can be identified in urine. The metabolite lactate has been identified by the present inventors in both serum and urine and is thus classed as both a blood metabolite and a urine metabolite.

The present inventors have discovered that results having high utility (such as high accuracy and high predictive value) require the concentrations of at least two metabolites to be determined. Prior art methods that measure only a single metabolite have been found to lack accuracy and predictive value.

The concentrations of the metabolites in the sample (as described above) can be determined using any suitable technique known in the art. By way of example, the following techniques can be used to detect and quantify small molecules in solution, and are thus suitable for determining metabolite concentrations: Nuclear Magnetic Resonance (NMR) spectroscopy, mass spectrometry, gas chromatography, ultraviolet (UV) spectrometry (for example in combination with high-performance liquid chromatography [HPLC] as HPLC-UV), and infrared spectroscopy.

In one embodiment, the concentrations of said metabolites are determined using NMR spectroscopy. In one embodiment, the concentrations of said metabolites are determined using mass spectrometry. In one embodiment, the concentrations of said metabolites are determined using HPLC-UV. In one embodiment, the concentrations of said metabolites are determined using infrared spectroscopy.

The concentration of a metabolite in the diagnostic sample can be expressed in a number of different ways, for example as molar concentration (number of moles of metabolite per unit volume of diagnostic sample) or mass concentration (mass of metabolite per unit volume of diagnostic sample). Alternatively, the concentration of a metabolite can be expressed as parts per million (ppm) or parts per billion (ppb). Such ways of expressing the concentration of a small molecule in solution are known in the art.

Thus, in one embodiment, the concentration of a metabolite in the diagnostic sample is the molar concentration of said metabolite. In one embodiment, the concentration of a metabolite in the diagnostic sample is the mass concentration of said metabolite.

The concentration of a metabolite in the diagnostic sample can be expressed by comparison to the concentration of a different metabolite in the same sample (i.e. in relative terms). By way of example, the concentration of a metabolite in the diagnostic sample in the diagnostic sample can be normalised by comparison to the concentration of a different reference metabolite within the same diagnostic sample. Examples of suitable reference metabolites are glucose and creatinine.

In one embodiment, a reference standard comprises (or consists of) a blood sample or samples (including a serum sample or samples and a plasma sample or samples), or a urine sample or samples, derived from blood or urine (as appropriate) obtained from a reference subject or subjects, wherein the reference subject is a subject other than the test subject.

In one embodiment, a "reference standard" comprises (or consists of) a set of data relating to the concentration of said metabolites in a blood (including serum and plasma) or urine sample or samples derived from blood or urine obtained from a reference subject or subjects, wherein the reference subject is a subject other than the test subject. The set of data (as described above) is derived by measuring the concentration of said metabolites. Said measuring may be carried out using any suitable technique known in the art, for example one of the following: Nuclear Magnetic Resonance (NMR) spectroscopy, mass spectrometry, gas chromatography, ultraviolet (UV) spectrometry (for example in combination with high-performance liquid chromatography [HPLC] as HPLC-UV), and infrared spectroscopy.

In one embodiment, the reference standard comprises (or consists of) a set of data relating to the concentration of said metabolites in a sample or samples derived from a single reference subject, wherein the reference subject is a subject other than the test subject

In one embodiment, the reference standard comprises (or consists of) a set of data relating to the concentration of said metabolites in a sample derived from a plurality of (i.e. two or more) reference subjects. Thus, in one embodiment, the reference standard is derived by pooling data obtained from two or more (e.g. three, four, five, 10, 15, 20 or 25) reference subjects and calculating an average (for example, mean or median) concentration for each metabolite. Thus, the reference standard may reflect average concentrations of said metabolites in blood and/or urine in a given population of reference subjects. Said concentrations may be expressed in relative terms, in the same manner as described above in relation to the sample that is to be tested using the method of the invention.

When comparing concentrations between the sample and the reference standard, the way in which the concentrations are expressed is matched between the sample and the reference standard. Thus, a relative concentration can be compared with a relative concentration.

The reference standard is derived from a subject (or subjects) that does not (or do not) have MS. Thus, comparison of a sample from a test subject with such a reference standard can permit the diagnosis of MS.

A reference standard derived from a subject (or subjects) having one of the following specific MS disease phases: secondary progressive, relapsing remitting, and primary progressive is described herein. A reference standard that is a specific MS disease phase standard is described herein.

A reference standard that is a secondary progressive phase MS standard is described herein. A reference standard that is derived from a subject or subjects previously diagnosed with secondary progressive phase MS is described herein.

A reference standard that is a relapsing remitting phase MS standard is described herein. A reference standard that is derived from a subject or subjects previously diagnosed with relapsing remitting phase MS is described herein.

The reference standard is derived from the same biological fluid type (blood or urine) as the sample that is being tested, thus allowing for an appropriate comparison between the two. Thus, by way of example, if the sample is derived from urine, the reference standard is also derived from urine. Alternatively, if the sample is a blood sample (e.g. a plasma or a serum sample), then the reference standard will also be a blood sample (e.g. a plasma sample or a serum sample, as appropriate).

The present inventors have discovered that variations in blood and/or urine concentrations of metabolites (as described above) can be identified in subjects having MS, as compared with healthy control subjects. Thus, said variations can be used to diagnose MS.

The term "concentration difference" embraces both positive and negative differences. Thus, a concentration difference can mean that the concentration of a metabolite is higher in the sample being tested than in the reference standard. Alternatively, a concentration difference can mean that the concentration of a metabolite is lower in the sample than in the reference standard.

The identification of a concentration difference (as described above) can be achieved using methods of statistical analysis. By way of example, NMR spectroscopy can be used to obtain an NMR spectrum for a sample. Methods of statistical analysis (for example, partial least squares discriminate analysis [PLS-DA]), can then be applied to compare said spectrum to an NMR spectrum obtained for a reference standard, allowing the identification of concentration differences.

It is described herein that variations in metabolite concentrations (as described above) can also be associated with specific MS disease phases (such as SP MS, RR MS, and PP MS). Thus, diagnosis of a specific MS disease phase in a test subject is described herein. This can be achieved both in subjects who have not previously been diagnosed with MS and in subjects who have previously been diagnosed with MS.

Thus, in permitting the diagnosis of a disease phase of MS in a test subject, the method described herein provides a significant advantage over prior art methods. Prior to the present disclosure, diagnosis of a disease phase of MS would be achieved retrospectively, through assessment of the clinical phenotype of an MS patient. The method described herein allows a much more rapid diagnosis of the disease phase of MS in a patient. By way of example, the method described herein permits diagnosis of a disease phase of MS in the absence of outward clinical signs of said phenotype. Thus, by way of further example, the method described herein allows the early diagnosis of secondary progressive phase MS in a patient previously diagnosed with relapsing remitting phase MS, where the patient has yet to manifest the outward clinical signs of secondary progressive MS. The method described herein therefore advantageously enables medical professionals to make improved choices regarding therapy options for an MS patient, and at an earlier stage than would otherwise be possible.

It is described herein that a specific MS disease phase can be diagnosed with increased accuracy using specific subsets of the metabolites listed above.

Metabolites comprising two or more blood metabolites (for example, two, three or more, four or more, five or more, six or more, seven or more, or eight or more) selected from: n-butyrate, N-acetyl aspartate, glucose, taurinebetaine, ascorbic acid, N-acetyl glycoprotein, fatty acid, and choline are described herein.

Metabolites comprising (or consisting of) the following eight blood metabolites: n-butyrate, N-acetyl aspartate, glucose, taurinebetaine, ascorbic acid, N-acetyl glycoprotein, fatty acid, and choline are described herein.

It is described herein that wherein said metabolites comprise two or more blood metabolites selected from: n-butyrate, N-acetyl aspartate, glucose, taurinebetaine, ascorbic acid, N-acetyl glycoprotein, fatty acid, and choline (as described above); the reference standard is derived from a subject (or subjects) that does not (or do not) have MS; and said concentration differences are selected from:
an increase in the concentration of one or more blood metabolites selected from: n-butyrate, N-acetyl aspartate, N-acetyl glycoprotein, and fatty acid; and/or a decrease in the concentration of one or more blood metabolites selected from: glucose, taurinebetaine, ascorbic acid, and choline.

Concentration differences comprising (or consisting of):
an increase in the concentration of: n-butyrate, N-acetyl aspartate, N-acetyl glycoprotein, and fatty acid; and a decrease in the concentration of: glucose, taurinebetaine, ascorbic acid, and choline are described herein.

It is described herein that changes in the concentration of the above described blood metabolites are suitable for use in diagnosing PP phase MS. Thus, in one description, wherein said metabolites comprise two or more blood metabolites selected from: n-butyrate, N-acetyl aspartate, glucose, taurinebetaine, ascorbic acid, N-acetyl glycoprotein, fatty acid, and choline (as described above); said concentration differences confirm the presence of primary progressive (PP) phase MS.

Metabolites comprising two or more blood metabolites (for example, two, three or more, four or more, five or more, or six) selected from: phosphocholine, lactate, N-acetyl species, beta-hydroxybutyrate, fatty acid (preferably fatty acid δ_{x-y}0.88, 1.30, 5.35), and glucose are described herein.

Metabolites comprising (or consisting of) the following six blood metabolites: phosphocholine, lactate, N-acetyl species, beta-hydroxybutyrate, fatty acid (preferably fatty acid δ_{x-y}0.88, 1.30, 5.35), and glucose are described herein.

It is described herein that, where said metabolites comprise two or more blood metabolites selected from: phosphocholine, lactate, N-acetyl species, beta-hydroxybutyrate, fatty acid (preferably fatty acid δ_{x-y}0.88, 1.30, 5.35), and glucose (as described above), the reference standard is derived from a subject (or subjects) that does not (or do not) have MS; and said concentration differences are selected from: an increase in the concentration of one or more blood metabolites selected from: fatty acid, N-acetyl species, and lactate;
and/or
a decrease in the concentration of one or more blood metabolites selected from: glucose, and phosphocholine.

Metabolites comprising two or more blood metabolites (for example, two, three or more, or four or more) selected from: fatty acid, N-acetyl species, glucose, phosphocholine, and L-glutamine are described herein.

Two or more blood metabolites further comprising a singlet (δ_{x-y}3.37) and a singlet (δ_{x-y}2.65) as measured using ¹H-NMR are described herein.

Metabolites comprising (or consisting of) the following five blood metabolites: fatty acid, N-acetyl species, glucose, phosphocholine, and L-glutamine are described herein.

It is described herein that, wherein said metabolites comprise two or more blood metabolites selected from: fatty acid, N-acetyl species, glucose, phosphocholine, and L-glutamine (as described above); the reference standard is derived from a subject (or subjects) that does not (or do not) have MS; and said concentration differences are selected from:
an increase in the concentration of one or more blood metabolites selected from: fatty acid, N-acetyl species, and L-glutamine; and/or a decrease in the concentration of one or more blood metabolites selected from: glucose, and phosphocholine.

Concentration differences comprising (or consisting of):
an increase in the concentration of: fatty acid, N-acetyl species, and L-glutamine; and a decrease in the concentration of: glucose, and phosphocholine are described herein.

Concentration differences comprising (or consisting of):
an increase in the concentration of: fatty acid, N-acetyl species, L-glutamine, and a singlet (δ_{x-y}3.37) as measured using ¹H-NMR; and a decrease in the concentration of: glucose, phosphocholine, and a singlet (δ_{x-y}2.65) as measured using ¹H-NMR are described herein.

Changes in the concentration of the above described blood metabolites that are particularly suitable for use in diagnosing PP phase MS are described herein. It is described herein that, wherein said metabolites comprise two or more blood metabolites selected from: phosphocholine, lactate, N-acetyl species, beta-hydroxybutyrate, fatty acid (preferably fatty acid δ_{x-y}0.88, 1.30, 5.35), and glucose; or fatty acid, N-acetyl species, glucose, phosphocholine, and L-glutamine (as described above); said concentration differences confirm the presence of primary progressive (PP) phase MS.

It is described herein that, wherein said metabolites comprise two or more blood metabolites selected from: fatty acid, N-acetyl species, glucose, phosphocholine, L-glutamine, a singlet (δ_{x-y}3.37) and a singlet (δ_{x-y}2.65) as measured using ¹H-NMR, (as described above); said concentration differences confirm the presence of primary progressive (PP) phase MS.

Metabolites comprising two or more blood metabolites (for example, two, three or more, four or more, five or more, six or more, seven or more, eight or more, or nine or more) selected from: fatty acid, ascorbic acid, n-butyrate, tyrosine, glucose, lactate, alanine, phosphocholine, oxyglutaric acid, and N-acetyl aspartate; and/or two or more urine metabolites (for example, two, or three or more) selected from: citrate, inositol, lactate, and TMAO are described herein.

Metabolites comprising two or more blood metabolites (for example, two, three or more, or four or more) selected from: fatty acid, ascorbic acid, n-butyrate, tyrosine and glucose are described herein.

Metabolites comprising (or consisting of) the following five blood metabolites: fatty acid, ascorbic acid, n-butyrate, tyrosine and glucose are described herein.

It is described herein that, wherein said metabolites comprise two or more blood metabolites selected from: fatty acid, ascorbic acid, n-butyrate, tyrosine and glucose (as described above); the reference standard is derived from a subject (or subjects) that does not (or do not) have MS, and said concentration differences are selected from:
a decrease in the concentration of one or more blood metabolites selected from: fatty acid, ascorbic acid, n-butyrate, tyrosine, and glucose.

Concentration differences comprising (or consisting of):
a decrease in the concentration of: fatty acid, ascorbic acid, n-butyrate, tyrosine, and glucose are described herein.

It is described herein that changes in the concentration of the above described blood metabolites are suitable for use in diagnosing SP phase MS. In one description wherein said metabolites comprise two or more blood metabolites selected from: fatty acid, ascorbic acid, n-butyrate, tyrosine and glucose (as described above); said concentration differences confirm the presence of secondary progressive (SP) phase MS.

Metabolites comprising two or more (for example, two, or three or more) urine metabolites selected from: citrate, inositol, lactate, and TMAO are described herein.

Metabolites comprising (or consisting of) the following four urine metabolites: citrate, inositol, lactate, and TMAO are described herein.

It is described herein that, wherein said metabolites comprise two or more urine metabolites selected from: citrate, inositol, lactate, and TMAO; the reference standard is derived from a subject (or subjects) that does not (or do not) have MS, and said concentration differences are selected from:
an increase in the concentration of one or more urine metabolites selected from: TMAO, and citrate; and/or a decrease in the concentration of one or more urine metabolites selected from: inositol, and lactate.

Concentration differences comprising (or consisting of):
an increase in the concentration of: TMAO, and citrate; and a decrease in the concentration of: inositol, and lactate are described herein.

It is described herein that changes in the concentration of the above described urine metabolites are suitable for use in diagnosing SP phase MS. In one description, wherein said metabolites comprise two or more urine metabolites selected from: citrate, inositol, lactate, and TMAO; said concentration differences confirm the presence of secondary progressive (SP) phase MS.

Metabolites comprising two or more blood metabolites (for example, two, three or more, four or more, five or more, six or more, or seven or more) selected from: n-butyrate, fatty acid, lactate, alanine, phosphocholine, oxyglutaric acid, N-acetyl aspartate, and glucose are described herein.

Metabolites comprising (or consisting of) the following eight blood metabolites: n-butyrate, fatty acid, lactate, alanine, phosphocholine, oxyglutaric acid, N-acetyl aspartate, and glucose are described herein.

It is described herein that, wherein said metabolites comprise two or more blood metabolites selected from: n-butyrate, fatty acid, lactate, alanine, phosphocholine, oxyglutaric acid, N-acetyl aspartate, and glucose (as described above); the reference standard is a relapsing remitting (RR) phase MS standard, and said concentration differences are selected from:
an increase in the concentration of one or more blood metabolites selected from: n-butyrate, fatty acid, lactate, and oxyglutaric acid; and a decrease in the concentration of one or more blood metabolites selected from: alanine, phosphocholine, N-acetyl aspartate, and/or glucose.

Concentration differences comprising (or consisting of):
an increase in the concentration of: n-butyrate, fatty acid, lactate, and oxyglutaric acid; and a decrease in the concentration of: alanine, phosphocholine, N-acetyl aspartate, and glucose are described herein.

It is described herein that changes in the concentration of the above described blood metabolites are particularly suitable for use in diagnosing SP phase MS. In one description wherein said metabolites comprise two or more blood metabolites selected from: n-butyrate, fatty acid, lactate, alanine, phosphocholine, oxyglutaric acid, N-acetyl aspartate, and glucose (as described above); said concentration differences confirm the presence of secondary progressive (SP) phase MS.

Metabolites comprising two or more blood metabolites (for example, two, three or more, four or more, five or more, or six or more) selected from: phosphocholine, lactate, N-acetyl species, and beta-hydroxybutyrate; or phosphocholine, lactate, N-acetyl species, beta-hydroxybutyrate, fatty acid, and glucose; or glucose, phosphocholine, fatty acid, lactate, alanine, beta-hydroxybutyrate, and N-acetyl species; and/or two or more urine metabolites (for example, two, or three or more) selected from: TMAO, citrate, creatinine, and lactate are described herein.

Two or more blood metabolites further comprising a singlet (δ_{x-y}3.37) as measured using ¹H-NMR are described herein.

Metabolites comprising two or more blood metabolites (for example, two, or three or more), selected from: fatty acid, glucose, phosphocholine, lactate, and N-acetyl species; or glucose, phosphocholine, fatty acid, and lactate are described herein.

Metabolites comprising (or consisting of) the following four blood metabolites: glucose, phosphocholine, fatty acid, and lactate are described herein.

It is described herein that, wherein said metabolites comprise two or more blood metabolites selected from: glucose, phosphocholine, fatty acid, and lactate (as described above); the reference standard is derived from a subject (or subjects) that does not (or do not) have MS, and said concentration differences are selected from:
a decrease in the concentration of one or more blood metabolites selected from:
   phosphocholine, lactate, N-acetyl species, and glucose; or
   a decrease in the concentration of one or more blood metabolites selected from: glucose, phosphocholine, fatty acid, and lactate.

Concentration differences comprising (or consisting of):
a decrease in the concentration of: glucose, phosphocholine, fatty acid, and lactate are described herein.

Concentration differences comprising (or consisting of): an increase in the concentration of a singlet (δ_{x-y}3.37) as measured using ¹H-NMR; and a decrease in the concentration of: glucose, phosphocholine, fatty acid, and lactate are described herein.

It is described herein that changes in the concentration of the above described blood metabolites are particularly suitable for use in diagnosing SP phase MS. Thus, in one description wherein said metabolites comprise two or more blood metabolites selected from: fatty acid, glucose, phosphocholine, lactate, and N-acetyl species; or glucose, phosphocholine, fatty acid, and lactate (as described above); said concentration differences confirm the presence of secondary progressive (SP) phase MS.

It is described herein that wherein said metabolites comprise two or more blood metabolites selected from: glucose, phosphocholine, fatty acid, lactate, and a singlet (δ_{x-y}3.37) as measured using ¹H-NMR (as described above); said concentration differences confirm the presence of secondary progressive (SP) phase MS.

In one embodiment, said metabolites comprise two or more (for example, two, or three or more) urine metabolites selected from: TMAO, citrate, creatinine, and lactate.

In one embodiment, said metabolites comprise (or consist of) the following four urine metabolites: TMAO, citrate, creatinine, and lactate.

In one embodiment, wherein said metabolites comprise two or more urine metabolites selected from: TMAO, citrate, creatinine, and lactate; the reference standard is derived from a subject (or subjects) that does not (or do not) have MS, and said concentration differences are selected from:
an increase in the concentration of one or more urine metabolites selected from: TMAO, and citrate; and/or a decrease in the concentration of one or more urine metabolites selected from: creatinine, and lactate.

In one embodiment, said concentration differences comprise (or consist of):
an increase in the concentration of: TMAO, and citrate; and a decrease in the concentration of: creatinine, and lactate.

It is described herein that changes in the concentration of the above described urine metabolites are particularly suitable for use in diagnosing SP phase MS. It is described herein that, wherein said metabolites comprise two or more urine metabolites selected from: TMAO, citrate, creatinine, and lactate; said concentration differences confirm the presence of secondary progressive (SP) phase MS.

Metabolites comprising two or more blood metabolites (for example, two, three or more, four or more, five or more, or six or more) selected from: phosphocholine, N-acetyl species, and beta-hydroxybutyrate; or phosphocholine, N-acetyl species, beta-hydroxybutyrate, fatty acid, and glucose; or fatty acid, lactate, alanine, phosphocholine, beta-hydroxybutyrate, N-acetyl species, and glucose are described herein.

Metabolites comprising (or consisting of) the following seven blood metabolites: fatty acid, lactate, alanine, phosphocholine, beta-hydroxybutyrate, N-acetyl species, and glucose are described herein.

It is described herein that, wherein said metabolites comprise two or more blood metabolites selected from: phosphocholine, N-acetyl species, and beta-hydroxybutyrate; or phosphocholine, N-acetyl species, beta-hydroxybutyrate, fatty acid, and glucose; or fatty acid, lactate, alanine, phosphocholine, beta-hydroxybutyrate, N-acetyl species, and glucose (as described above); the reference standard is a relapsing remitting (RR) phase MS standard, and said concentration differences are selected from:
an increase in the concentration of: beta-hydroxybutyrate; and/or a decrease in the concentration of one or more blood metabolites selected from: phosphocholine, N-acetyl species, and glucose, and optionally fatty acid (preferably fatty acid δ_{x-y}0.88, 1.30, 5.35); or said concentration differences are selected from:
   an increase in the concentration of one or more blood metabolites selected from: fatty acid, lactate, and beta-hydroxybutyrate; and a decrease in the concentration of one or more blood metabolites selected from: alanine, phosphocholine, N-acetyl species, and glucose.

Concentration differences comprising (or consisting of):
an increase in the concentration of: beta-hydroxybutyrate; and a decrease in the concentration of one or more blood metabolites selected from: phosphocholine, N-acetyl species, and glucose, and optionally fatty acid (preferably fatty acid δ_{x-y}0.88, 1.30, 5.35); or an increase in the concentration of: fatty acid, lactate, and beta-hydroxybutyrate; and a decrease in the concentration of: alanine, phosphocholine, N-acetyl species, and glucose are described herein.

It is described herein that changes in the concentration of the above described blood metabolites are particularly suitable for use in diagnosing SP phase MS. In one description wherein said metabolites comprise two or more blood metabolites selected from phosphocholine, N-acetyl species, and beta-hydroxybutyrate; or phosphocholine, N-acetyl species, beta-hydroxybutyrate, fatty acid, and glucose; or fatty acid, lactate, alanine, phosphocholine, beta-hydroxybutyrate, N-acetyl species, and glucose (as described above); said concentration differences confirm the presence of secondary progressive (SP) phase MS.

Metabolites comprising two or more blood metabolites (for example, two, three or more, four or more, five or more, six or more, seven or more, eight or more, nine or more, 10 or more, or 11 or more) selected from: N-acetyl glycoprotein, phosphocholine, glucose, taurinebetaine, ascorbic acid, fatty acid, n-butyrate, tyrosine, N-acetyl aspartate, lactate, alanine, and oxyglutaric acid are described herein.

Metabolites comprising two or more blood metabolites (for example, two, three or more, four or more, five or more, six or more, seven or more, or eight or more) selected from: N-acetyl glycoprotein, phosphocholine, glucose, taurinebetaine, ascorbic acid, fatty acid, n-butyrate, tyrosine, and N-acetyl aspartate are described herein.

Metabolites comprising (or consisting of) the following nine blood metabolites: N-acetyl glycoprotein, phosphocholine, glucose, taurinebetaine, ascorbic acid, fatty acid, n-butyrate, tyrosine, and N-acetyl aspartate are described herein.

It is described herein that, wherein said metabolites comprise two or more metabolites selected from: N-acetyl glycoprotein, phosphocholine, glucose, taurinebetaine, ascorbic acid, fatty acid, n-butyrate, tyrosine, and N-acetyl aspartate; the reference standard is derived from a subject (or subjects) that does not (or do not) have MS; and said concentration differences are selected from:
an increase in the concentration of one or more blood metabolites selected from: N-acetyl glycoprotein, phosphocholine, fatty acid, n-butyrate and/or tyrosine; and/or a decrease in the concentration of one or more blood metabolites selected from: glucose, taurinebetaine, ascorbic acid, and N-acetyl aspartate.

Concentration differences comprising (or consisting of):
an increase in the concentration of: N-acetyl glycoprotein, phosphocholine, fatty acid, n-butyrate and tyrosine; and a decrease in the concentration of: glucose, taurinebetaine, ascorbic acid, and N-acetyl aspartate are described herein.

Metabolites comprising two or more blood metabolites (for example, two, three or more, four or more, five or more, six or more, or seven or more) selected from: n-butyrate, fatty acid, lactate, alanine, phosphocholine, oxyglutaric acid, N-acetyl aspartate, and glucose are described herein.

Metabolites comprising (or consisting of): n-butyrate, fatty acid, lactate, alanine, phosphocholine, oxyglutaric acid, N-acetyl aspartate, and glucose are described herein.

It is described herein that, wherein said metabolites comprise two or more metabolites selected from: n-butyrate, fatty acid, lactate, alanine, phosphocholine, oxyglutaric acid, N-acetyl aspartate, and glucose; the reference standard is a secondary progressive (SP) phase MS standard, and said concentration differences are selected from:
an increase in the concentration of one or more blood metabolites selected from: alanine, phosphocholine, N-acetyl aspartate, and glucose; and a decrease in the concentration of: n-butyrate, fatty acid, lactate, and/or oxyglutaric acid.

Concentration differences comprising (or consisting of) an increase in the concentration of: alanine, phosphocholine, N-acetyl aspartate, and glucose; and a decrease in the concentration of: n-butyrate, fatty acid, lactate, and oxyglutaric acid are described herein.

It is described herein that, wherein said metabolites comprise at least two blood metabolites selected from: N-acetyl glycoprotein, phosphocholine, glucose, taurinebetaine, ascorbic acid, fatty acid, n-butyrate, tyrosine, N-acetyl aspartate, lactate, alanine, and oxyglutaric acid (as described above); said concentration differences confirm the presence of relapsing remitting (RR) phase MS.

Metabolites comprising two or more blood metabolites (for example, two, three or more, four or more, five or more, six or more, or seven or more) selected from: phosphocholine, N-acetyl species, beta-hydroxybutyrate, and lactate; or phosphocholine, N-acetyl species, beta-hydroxybutyrate, lactate, fatty acid, and glucose; or L-glutamine, alanine, glucose, phosphocholine, lactate, fatty acid, beta-hydroxybutyrate, and N-acetyl species are described herein.

Metabolites comprising two or more blood metabolites (for example, two, three or more, or four or more) selected from: L-glutamine, alanine, glucose, phosphocholine, and lactate are described herein.

Metabolites comprising (or consisting of) the following five blood metabolites: L-glutamine, alanine, glucose, phosphocholine, and lactate are described herein.

It is described herein that, wherein said metabolites comprise two or more metabolites selected from: phosphocholine, N-acetyl species, beta-hydroxybutyrate, and lactate; or phosphocholine, N-acetyl species, beta-hydroxybutyrate, lactate, fatty acid, and glucose; or L-glutamine, alanine, glucose, phosphocholine, and lactate; the reference standard is derived from a subject (or subjects) that does not (or do not) have MS; and said concentration differences are selected from:
a decrease in the concentration of one or more blood metabolites selected from: glucose, phosphocholine, lactate, and N-acetyl species;
or an increase in the concentration of one or more blood metabolites selected from: L-glutamine, and alanine; and/or a decrease in the concentration of one or more blood metabolites selected from: glucose, phosphocholine, and lactate.

Concentration differences comprising (or consisting of):
an increase in the concentration of: a singlet (δ_{x-y}3.37) as measured using ¹H-NMR, L-glutamine, and alanine; and a decrease in the concentration of: glucose, phosphocholine, and lactate are described herein.

Concentration differences comprising (or consisting of):
a decrease in the concentration of one or more blood metabolites selected from: glucose, phosphocholine, lactate, and N-acetyl species;
or an increase in the concentration of: L-glutamine, and alanine; and a decrease in the concentration of: glucose, phosphocholine, and lactate are described herein.

Metabolites comprising two or more blood metabolites (for example, two, three or more, four or more, five or more, or six or more) selected from: fatty acid, lactate, alanine, phosphocholine, beta-hydroxybutyrate, N-acetyl species, and glucose are described herein.

Metabolites comprising (or consisting of): fatty acid, lactate, alanine, phosphocholine, beta-hydroxybutyrate, N-acetyl species, and glucose are described herein.

It is described herein that, wherein said metabolites comprise two or more metabolites selected from: fatty acid, lactate, alanine, phosphocholine, beta-hydroxybutyrate, N-acetyl species, and glucose; the reference standard is a secondary progressive (SP) phase MS standard, and said concentration differences are selected from:
an increase in the concentration of one or more blood metabolites selected from: alanine, phosphocholine, N-acetyl species, and glucose; and a decrease in the concentration of: fatty acid, lactate, and beta-hydroxybutyrate.

Concentration differences comprising (or consisting of) an increase in the concentration of: alanine, phosphocholine, N-acetyl species, and glucose; and a decrease in the concentration of: fatty acid, lactate, and beta-hydroxybutyrate are described herein.

It is described herein that, wherein said metabolites comprise at least two blood metabolites selected from: phosphocholine, N-acetyl species, beta-hydroxybutyrate, and lactate; or phosphocholine, N-acetyl species, beta-hydroxybutyrate, lactate, fatty acid, and glucose; or L-glutamine, alanine, glucose, phosphocholine, lactate, fatty acid, beta-hydroxybutyrate, and N-acetyl species (as described above); said concentration differences confirm the presence of relapsing remitting (RR) phase MS.

A method for diagnosing Multiple Sclerosis in a human subject by measuring the concentration of two or more metabolites using NMR spectroscopy is described herein.

Described herein is an *in vitro* method for diagnosing MS in a human test subject using ¹H-NMR spectroscopy, comprising
(i) determining the concentrations of two or more metabolites in a sample from said subject, wherein said two or more metabolites are selected from:
   blood metabolites, wherein said blood metabolites comprise (or consist of) chemical species having the following parameters as measured by ¹H-NMR, with chemical shifts defined relative to the methyl resonance of 3-trimethylsilyl-1-propionate (TSP) defined at 0.00 ppm: (δ1.46, d, C*H*₃; δ3.73, q, C*H*), (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ1.32 d, C*H*₃, δ4.11, q, C*H*), (δ3.25, s, N(C*H*₃)₃, δ3.59, t, NC*H*₂, δ4.17, m, NCH₂C*H*₂), (δ2.13, m, C*H*₂CHNH₂, δ2.45, m, OCC*H*₂, δ3.77, t, CH₂C*H*NH₂), (δ2.03, br s, COC*H*₃), (δ1.19 d, C*H*₃, δ2.28, m, C*H*_{A}H_{B}, δ2.37, m, CH_{A}*H*_{B}, δ4.12, m, CH), (δ_{x-y}3.37), and (δ_{x-y}2.65);
      and/or
   urine metabolites, wherein said urine metabolites comprise (or consist of) chemical species having the following parameters as measure by ¹H-NMR, with chemical shifts defined relative to the methyl resonance of 3-trimethylsilyl-1-propionate (TSP) defined at 0.00 ppm: (δ2.50, d, C*H*_{A}H_{B}, δ2.65, d, CH_{A}*H*_{B}), (δ3.05, s, NC*H*₃, δ4.10, s, C*H*₂), (δ1.32 d, C*H*₃, δ4.11, q, C*H*) and (δ3.26, s, ON(C*H*₃)₃);
(ii) comparing the concentrations of said two or more metabolites in the sample with the concentrations of the same metabolites in at least one reference standard; and
(iii) identifying a concentration difference for each of said two or more metabolites in the sample relative to the reference standard;
wherein said concentration differences correlate with the presence of MS.

Described herein are two or more metabolites selected from: blood metabolites, wherein said blood metabolites comprise: (δ2.13, m, C*H*₂CHNH₂, δ2.45, m, OCC*H*₂, δ3.77, t, CH₂C*H*NH₂), (δ1.46, d, C*H*₃; δ3.73, q, C*H*), (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃)₃, (δ0.90, br, C*H*₃, δ1.30 br, C*H*₂CH₂CH₃, δ1.55 br, C*H*₂CH₃, δ2.05, br, C*H*₂CH=, δ2.25, br, C*H*₂COOH, δ5.35, br, CH₂C*H*=), (δ2.03, br s, COC*H*₃), (δ1.32 d, C*H*₃, δ4.11, q, C*H*), and(δ1.19, d, C*H*₃, δ2.28, m, C*H*_{A}H_{B}, δ2.37, m, CH_{A}*H*_{B}, δ4.12, m, C*H*); and/or urine metabolites, wherein said urine metabolites comprise: (δ3.26, s, ON(C*H*₃)₃), (δ2.50, d, C*H*_{A}H_{B}, δ2.65, d, CH_{A}*H*_{B}), (δ3.05, s, NC*H*₃, δ4.10, s, C*H*₂), and(δ1.32, d, C*H*₃, δ4.11, q, C*H*).

Blood metabolites further comprising a singlet (δ_{x-y}3.37) and a singlet (δ_{x-y}2.65) as measured using ¹H-NMR are described herein.

Metabolites comprising two or more blood metabolites (for example, two, three or more, or four or more) selected from: (δ0.90, br, C*H*₃, δ1.30 br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ2.03, br s, COC*H*₃), (δ3.42, dd, C(4)*H*, δ3.44, dd, C(6)*H*_{A}H_{B}, δ3.54, dd, C(2)*H*, δ3.73, dd, C(3)*H,* δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), and (δ2.13, m, C*H*₂CHNH₂, δ2.45, m, OCC*H*₂, δ3.77, t, CH₂C*H*NH₂) are described herein.

Two or more blood metabolites further comprising a singlet (δ_{x-y}3.37) and a singlet (δ_{x-y}2.65) are described herein.

Metabolites comprising (or consisting of) the following five blood metabolites: (δ0.90, br, C*H*₃, δ1.30 br, C*H*₂CH₂CH₃, δ1.55 br, C*H*₂CH₃, δ2.05, br, C*H*₂CR=, δ2.25, br, C*H*₂COOH, δ5.35, br, CH₂C*H*=), (δ2.03, br s, COC*H*₃), (δ3.42, dd, C(4)*H*, δ3.44, dd, C(6)*H*_{A}H_{B}, δ3.54, dd, C(2)*H*, δ3.73, dd, C(3)H, δ3.79, dd, C(6)H_{A}*H*_{B}, δ3.86, m, C(5)*H,* δ5.24, d, C(1)*H*) and/or (δ3.26, dd, C(2)*H*, δ3.40, dd, C(4)*H*, δ3.47, dd, C(5)*H,* δ3.49, dd, C(3)*H*, δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), and(δ2.13, m, C*H*₂CHNH₂, δ2.45, m, OCC*H*₂, δ3.77, t, CH₂C*H*NH₂) are described herein.

It is described herein that, wherein said metabolites comprise two or more blood metabolites selected from: (δ0.90, br, C*H*₃, δ1.30 br, C*H*₂CH₂CH₃, δ1.55 br, C*H*₂CH₃, δ2.05, br, C*H*₂CH=, δ2.25, br, C*H*₂COOH, δ5.35, br, CH₂C*H*=), (δ2.03, br s, COC*H*₃), (δ3.42, dd,

C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), and (δ2.13, m, C*H*₂CHNH₂, δ2.45, m, OCC*H*₂, δ3.77, t, CH₂C*H*NH₂) (as described above); the reference standard is derived from a subject (or subjects) that does not (or do not) have MS; and said concentration differences are selected from:
an increase in the concentration of one or more blood metabolites selected from: (δ0.90, br, C*H*₃, δ1.30 br, C*H*₂CH₂CH₃, δ1.55 br, C*H*₂CH₃, δ2.05, br, C*H*₂CH=, δ2.25, br, C*H*₂COOH, δ5.35, br, CH₂C*H*=), (δ2.03, br s, COC*H*₃), and (δ2.13, m, C*H*₂CHNH₂, δ2.45, m, OCC*H*₂, δ3.77, t, CH₂C*H*NH₂); and/or a decrease in the concentration of one or more blood metabolites selected from: (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), and(δ3.25, s, N(C*H*₃)₃, δ3.59, t, NC*H*₂, δ4.17, m, NCH₂C*H*₂).

Concentration differences comprising (or consisting of):
an increase in the concentration of: (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ2.03, br s, COC*H*₃), and (δ2.13, m, C*H*₂CHNH₂, δ2.45, m, OCC*H*₂, δ3.77, t, CH₂C*H*NH₂); and a decrease in the concentration of: (δ3.42, dd, C(4)*H*, δ3.44, dd, C(6)*H*_{A}H_{B}, δ3.54, dd, C(2)H, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H,* δ3.40, dd, C(4)*H*, δ3.47, dd, C(5)*H,* δ3.49, dd, C(3)*H*, δ3.86, d, C(6)*H*₂, δ4.63, d, C(1)*H*), and (δ3.25, s, N(C*H*₃)₃, δ3.59, t, NC*H*₂, δ4.17, m, NCH₂C*H*₂) are described herein.

Concentration differences comprising (or consisting of):
an increase in the concentration of: (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ2.03, br s, COC*H*₃), (δ2.13, m, C*H*₂CHNH₂, δ2.45, m, OCC*H*₂, δ3.77, t, CH₂C*H*NH₂) and a singlet (δ_{x-y}3.37); and a decrease in the concentration of: (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), and a singlet (δ_{x-y}2.65) are described herein.

It is described herein that changes in the concentration of the above described blood metabolites are particularly suitable for use in diagnosing PP phase MS. Thus, in one embodiment, wherein said metabolites comprise two or more blood metabolites selected from: (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ2.03, br s, COC*H*₃), (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H,* δ3.49, dd, C(3)*H*, δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), and(δ2.13, m, C*H*₂CHNH₂, δ2.45, m, OCC*H*₂, δ3.77, t, CH₂C*H*NH₂). (as described above); said concentration differences confirm the presence of primary progressive (PP) phase MS.

It is described herein that, wherein said metabolites comprise two or more blood metabolites selected from: (δ0.90, br, C*H*₃, δ1.30 br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ2.03, br s, COC*H*₃), (δ3.42, dd, C(4)H, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H,* δ5.24, d, C(1)*H*) and/or (δ3.26, dd, C(2)*H*, δ3.40, dd, C(4)*H*, δ3.47, dd, C(5)*H*, δ3.49, dd, *C(3)H,* δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), (δ2.13, m, C*H*₂CHNH₂, δ2.45, m, OCC*H*₂, δ3.77, t, CH₂C*H*NH₂)., a singlet (δ_{x-y}3.37) and a singlet (δ_{x-y}2.65), (as described above); said concentration differences confirm the presence of primary progressive (PP) phase MS.

Metabolites comprising two or more blood metabolites (for example, two, three or more, four or more, five or more, or six or more) selected from: (δ3.42, dd, C(4)*H*, δ3.44, dd, C(6)*H*_{A}H_{B}, δ3.54, dd, C(2)*H*, δ3.73, dd, C(3)*H*, δ3.79, dd,

C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ1.32, d, C*H*₃, δ4.11, q, C*H*), (δ1.46, d, C*H*₃; δ3.73, q, C*H*), (δ1.19, d, C*H*₃, δ2.28, m, C*H*_{A}H_{B}, δ2.37, m, CH_{A}*H*_{B}, δ4.12, m, C*H*), and(δ2.03, br s, COC*H*₃); and/or two or more urine metabolites (for example, two, or three or more) selected from:(δ3.26, s, ON(C*H*₃)₃), (δ2.50, d, C*H*_{A}H_{B}, δ2.65, d, CH_{A}*H*_{B}), (δ3.05, s, NC*H*₃, δ4.10, s, C*H*₂), and(δ1.32, d, C*H*₃, δ4.11, q, C*H*) are described herein.

Two or more blood metabolites further comprising a singlet (δ_{x-y}3.37) are described herein.

Metabolites comprising two or more blood metabolites (for example, two, or three or more), selected from: (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃*)*₃*,* δ3.59, t, NC*H*₂, δ4.17, m, NCH₂C*H*₂), (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), and(δ1.32, d, C*H*₃, δ4.11, q, C*H*) are described herein.

Metabolites comprising (or consisting of) the following four blood metabolites: (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), and(δ1.32, d, C*H*₃, δ4.11, q, C*H*) are described herein.

It is described herein that, wherein said metabolites comprise two or more blood metabolites selected from: (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), (δ0.90, br, C*H*₃, δ1.30 br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), and(δ1.32, d, C*H*₃, δ4.11, q, C*H*) (as described above); the reference standard is derived from a subject (or subjects) that does not (or do not) have MS, and said concentration differences are selected from:
a decrease in the concentration of one or more blood metabolites selected from: (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), and(δ1.32, d, C*H*₃, δ4.11, q, C*H*).

Concentration differences comprising (or consisting of):
a decrease in the concentration of: (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H,* δ5.24, d, C(1)*H*) and/or (δ3.26, dd, C(2)*H*, δ3.40, dd, C(4)*H*, δ3.47, dd, C(5)*H,* δ3.49, dd, C(3)*H,* δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), and(δ1.32, d, C*H*₃, δ4.11, q, C*H*) are described herein.

Concentration differences comprising (or consisting of): an increase in the concentration of a singlet (δ_{x-y}3.37); and a decrease in the concentration of: :(δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd,

C(4)*H*, δ3.47, dd, C(5)*H*, δ3.49, dd, C(3)*H*, δ3.86, d, C(6)*H₂,* δ4.63, d, C(1)*H*), (δ3.25, s, N(C*H*₃)₃, δ3.59, t, NC*H*₂, δ4.17, m, NCH₂C*H*₂), (δ0.90, br, *CH*₃, δ1.30, br, C*H*₂CH₂CH₃, δ1.55 br, C*H*₂CH₃, δ2.05, br, C*H*₂CH=, δ2.25, br, C*H*₂COOH, δ5.35, br, CH₂C*H*=), and(δ1.32 d, C*H*₃, δ4.11, q, C*H*) are described herein.

It is described herein that changes in the concentration of the above described blood metabolites are particularly suitable for use in diagnosing SP phase MS. Thus, in one embodiment wherein said metabolites comprise two or more blood metabolites selected from: (δ3.42, dd, C(4)*H*, δ3.44, dd, C(6)*H*_{A}H_{B}, δ3.54, dd, C(2)*H*, δ3.73, dd, C(3)*H,* δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H₂*, δ4.63, d, C(1)*H*), (δ3.25, s, N(C*H*₃)₃, δ3.59, t, NC*H₂*, δ4.17, m, NCH₂C*H*₂), (δ0.90, br, C*H*₃*,* δ1.30, br, C*H*₂CH₂CH₃, δ1.55 br, C*H*₂CH₃, δ2.05, br, C*H*₂CH=, δ2.25, br, C*H*₂COOH, δ5.35, br, CH₂C*H*=), and(δ1.32, d, C*H*₃, δ4.11, q, C*H*) (as described above); said concentration differences confirm the presence of secondary progressive (SP) phase MS.

It is described herein that wherein said metabolites comprise two or more blood metabolites selected from: (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H,* δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H₂*, δ4.63, d, C(1)*H*), (δ3.25, s, N(C*H*₃)₃, δ3.59, t, NC*H*₂, δ4.17, m, NCH₂C*H*₂), (δ0.90, br, *CH*₃, δ1.30, br, C*H*₂C*H*₂CH₃, δ1.55, br, C*H*₂CH₃, δ2.05, br, C*H*₂CH=, δ2.25, br, C*H*₂COOH, δ5.35, br, CH₂C*H*=), (δ1.32 d, C*H*₃, δ4.11, q, C*H*), and a singlet (δ_{x-y}3.37) (as described above); said concentration differences confirm the presence of secondary progressive (SP) phase MS.

Metabolites comprising two or more (for example, two, or three or more) urine metabolites selected from: (δ3.26, s, ON(C*H*₃)₃), (δ2.50, d, C*H*_{A}H_{B}, δ2.65, d, CH_{A}*H*_{B}), (δ3.05, s, NC*H*₃, δ4.10, s, C*H*₂), and(δ1.32 d, C*H*₃, δ4.11, q, C*H*) are described herein. Metabolites comprising (or consisting of) the following four urine metabolites: (δ3.26, s, ON(C*H*₃)₃), (δ2.50, d, C*H*_{A}H_{B}, δ2.65, d, CH_{A}*H*_{B}), (δ3.05, s, NC*H*₃, δ4.10, s, C*H*₂), and(δ1.32 d, C*H*₃, δ4.11, q, C*H*) are described herein.

It is described herein that, wherein said metabolites comprise two or more urine metabolites selected from: (δ3.26, s, ON(C*H*₃)₃), (δ2.50, d, C*H*_{A}H_{B}, δ2.65, d, CH_{A}*H*_{B}), (δ3.05, s, NC*H*₃, δ4.10, s, C*H*₂), and(δ1.32, d, C*H*₃, δ4.11, q, C*H*); the reference standard is derived from a subject (or subjects) that does not (or do not) have MS, and said concentration differences are selected from:
an increase in the concentration of one or more urine metabolites selected from:(δ3.26, s, ON(C*H*₃)₃), and(δ2.50, d, C*H*_{A}H_{B}, δ2.65, d, CH_{A}*H*_{B}); and/or a decrease in the concentration of one or more urine metabolites selected from:(δ3.05, s, NC*H₃*, δ4.10, s, C*H*₂), and(δ1.32, d, C*H*₃, δ4.11, q, C*H*).

Concentration differences comprising (or consisting of):
an increase in the concentration of: (δ3.26, s, ON(C*H*₃)₃), and(δ2.50, d, C*H*_{A}H_{B}, δ2.65, d, CH_{A}*H*_{B}); and a decrease in the concentration of: (δ3.05, s, NC*H*₃, δ4.10, s, C*H*₂), and(δ1.32 d, C*H*₃, δ4.11, q, C*H*) are described herein.

It is described herein that changes in the concentration of the above described urine metabolites are particularly suitable for use in diagnosing SP phase MS. In one description, wherein said metabolites comprise two or more urine metabolites selected from: (δ3.26, s, ON(C*H*₃)₃), (δ2.50, d, C*H*_{A}H_{B}, δ2.65, d, CH_{A}*H*_{B}), (δ3.05, s, NC*H₃*, δ4.10, s, C*H*₂), and(δ1.32, d, C*H*₃, δ4.11, q, C*H*); said concentration differences confirm the presence of secondary progressive (SP) phase MS.

Metabolites comprising two or more blood metabolites (for example, two, three or more, four or more, five or more, or six or more) selected from: (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ1.32, d, C*H*₃, δ4.11, q, C*H*), (δ1.46, d, C*H*₃; δ3.73, q, C*H*), (δ3.25, s, N(C*H*₃)₃, δ3.59, t, NC*H*₂, δ4.17, m, NCH₂C*H*₂), (δ1.19, d, C*H*₃, δ2.28, m, C*H*_{A}H_{B}, δ2.37, m, CH_{A}*H*_{B}, δ4.12, m, C*H*), (δ2.03, br s, COC*H*₃), and(δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*) are described herein.

Metabolites comprising (or consisting of) the following seven blood metabolites: (δ0.90, br, C*H*₃, δ1.30 br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ1.32 d, C*H*₃, δ4.11, q, C*H*), (δ1.46, d, C*H*₃; δ3.73, q, C*H*), (δ3.25, s, N(C*H*₃)₃, δ3.59, t, NC*H*₂, δ4.17, m, NCH₂C*H*₂), (δ1.19, d, C*H*₃, δ2.28, m, C*H*_{A}H_{B}, δ2.37, m, CH_{A}*H*_{B}, δ4.12, m, C*H*), (δ2.03, br s, COC*H*₃), and(δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H,* δ3.49, dd, C(3)*H*, δ3.86, d, C(6)*H*₂, δ4.63, d, C(1)*H*) are described herein.

It is described herein that, wherein said metabolites comprise two or more blood metabolites selected from: (δ0.90, br, C*H*₃, δ1.30, br, C*H*₂CH₂CH₃, δ1.55 br, C*H*₂CH₃, δ2.05, br, C*H*₂CH=, δ2.25, br, C*H*₂COOH, δ5.35, br, CH₂C*H*=), (δ1.32, d, C*H*₃, δ4.11, q, C*H*), (δ1.46, d, C*H*₃; δ3.73, q, C*H*), (δ3.25, s, N(C*H*₃)₃, δ3.59, t, NC*H*₂, δ4.17, m, NCH₂C*H*₂), (δ1.19, d, C*H*₃, δ2.28, m, C*H*_{A}H_{B}, δ2.37, m, CH_{A}*H*_{B}, δ4.12, m, C*H*), (δ2.03, br s, COC*H*₃), and(δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*) (as described above); the reference standard is a relapsing remitting (RR) phase MS standard, and said concentration differences are selected from:
an increase in the concentration of one or more blood metabolites selected from:(δ0.90, br, C*H*₃, δ1.30, br, C*H*₂CH₂CH₃, δ1.55, br, C*H*₂CH₃, δ82.05, br, C*H*₂CH=, δ2.25, br, C*H*₂COOH, δ5.35, br, CH₂C*H*=), (δ1.32, d, C*H*₃, δ4.11, q, C*H*), and(δ1.19, d, C*H*₃, δ2.28, m, C*H*_{A}H_{B}, δ2.37, m, CH_{A}*H*_{B}, δ4.12, m, C*H*); and a decrease in the concentration of one or more blood metabolites selected from:(δ1.46, d, C*H*₃; δ3.73, q, C*H*), (δ3.25, s, N(C*H*₃)₃, δ3.59, t, NC*H*₂, δ4.17, m, NCH₂C*H*₂), (δ2.03, br s, COC*H*₃), and(δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H₂*, δ4.63, d, C(1)*H*).

Concentration differences comprising (or consisting of):
an increase in the concentration of: (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ1.32, d, C*H*₃, δ4.11, q, C*H*), and(δ1.19, d, C*H*₃, δ2.28, m, C*H*_{A}H_{B}, δ2.37, m, CH_{A}*H*_{B}, δ4.12, m, C*H*); and a decrease in the concentration of: (δ1.46, d, C*H*₃; δ3.73, q, C*H*), (δ3.25, s, N(C*H*₃)₃, δ3.59, t, NC*H*₂, δ4.17, m, NCH₂C*H*₂), (δ2.03, br s, COC*H*₃), and(δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H,* δ3.49, dd, C(3)*H*, δ3.86, d, C(6)*H*₂, δ4.63, d, C(1)*H*) are described herein.

It is described herein that changes in the concentration of the above described blood metabolites are particularly suitable for use in diagnosing SP phase MS. In one embodiment wherein said metabolites comprise two or more blood metabolites selected from (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ1.32, d, C*H*₃, δ4.11, q, C*H*), (δ1.46, d, C*H*₃; δ3.73, q, C*H*), (δ3.25, s, N(C*H*₃)₃, δ3.59, t, NC*H₂*, δ4.17, m, NCH₂C*H*₂), (δ1.19, d, C*H*₃, δ2.28, m, C*H*_{A}H_{B}, δ2.37, m, CH_{A}*H*_{B}, δ4.12, m, C*H*), (δ2.03, br s, COC*H*₃), and(δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H₂*, δ4.63, d,
C(1)*H*) (as described above); said concentration differences confirm the presence of secondary progressive (SP) phase MS.

Metabolites comprising two or more blood metabolites (for example, two, three or more, four or more, five or more, six or more, or seven or more) selected from: (δ2.13, m, C*H*₂CHNH₂, δ2.45, m, OCC*H*₂, δ3.77, t, CH₂C*H*NH₂), (δ1.46, d, C*H*₃; δ3.73, q, C*H*), (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H₂*, δ4.63, d, C(1)*H*), (δ3.25, s, N(C*H*₃)₃, δ3.59, t, NC*H₂*, δ4.17, m, NCH₂C*H*₂), (δ1.32, d, C*H*₃, δ4.11, q, C*H*), (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ1.19, d, C*H*₃, δ2.28, m, C*H*_{A}H_{B}, δ2.37, m, CH_{A}*H*_{B}, δ4.12, m, C*H*), and(δ2.03, br s, COC*H*₃) are described herein.

Metabolites comprising two or more blood metabolites (for example, two, three or more, or four or more) selected from: (δ2.13, m, C*H*₂CHNH₂, δ2.45, m, OCC*H*₂, δ3.77, t, CH₂C*H*NH₂), (δ1.46, d, C*H*₃; δ3.73, q, C*H*), (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H,* δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), (δ1.32, d, C*H*₃, δ4.11, q, C*H*) are described herein.

Metabolites comprising (or consisting of) the following five blood metabolites: (δ2.13, m, C*H*₂CHNH₂, δ2.45, m, OCC*H*₂, δ3.77, t, CH₂C*H*NH₂), (δ1.46, d, C*H*₃; δ3.73, q, C*H*), (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), (δ1.32, d, C*H*₃, δ4.11, q, C*H*) are described herein.

It is described herein that, wherein said metabolites comprise two or more metabolites selected from: (δ2.13, m, C*H*₂CHNH₂, δ2.45, m, OCC*H*₂, δ3.77, t, CH₂C*H*NH₂), (δ1.46, d, C*H*₃; δ3.73, q, C*H*), (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), (δ1.32, d, C*H*₃, δ4.11, q, C*H*); the reference standard is derived from a subject (or subjects) that does not (or do not) have MS; and said concentration differences are selected from:
an increase in the concentration of one or more blood metabolites selected from:(δ2.13, m, C*H*₂CHNH₂, δ2.45, m, OCC*H*₂, δ3.77, t, CH₂C*H*NH₂), and(δ1.46, d, C*H*₃; δ3.73, q, C*H*); and/or a decrease in the concentration of one or more blood metabolites selected from:(δ₃.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃)₃, δ3.59, t, NC*H*₂, δ4.17, m, NCH₂C*H*₂), and(δ1.32, d, C*H*₃, δ4.11, q, C*H*).

Concentration differences comprising (or consisting of):
an increase in the concentration of: a singlet (δ_{x-y}3.37), (δ2.13, m, C*H*₂CHNH₂, δ2.45, m, OCC*H*₂, δ3.77, t, CH₂C*H*NH₂), and(δ1.46 d, C*H*₃; δ3.73, q, C*H*); and a decrease in the concentration of:(δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H₂*, δ4.63, d, C(1)*H*), (δ3.25, s, N(C*H*₃)₃, δ3.59, t, NC*H*₂, δ4.17, m, NCH₂C*H*₂), and(δ1.32, d, C*H*₃, δ4.11, q, C*H*) are described herein.

Concentration differences comprising (or consisting of):
an increase in the concentration of: (δ2.13, m, C*H*₂CHNH₂, δ2.45, m, OCC*H*₂, δ3.77, t, CH₂C*H*NH₂), and(δ1.46, d, C*H*₃; δ3.73, q, C*H*); and a decrease in the concentration of: (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*), (δ3.25, s, N(C*H*₃)₃, δ3.59, t, NC*H₂,* δ4.17, m, NCH₂C*H*₂), and(δ1.32, d, C*H*₃, δ4.11, q, C*H*) are described herein.

Metabolites comprising two or more blood metabolites (for example, two, three or more, four or more, five or more, or six or more) selected from: (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ1.32, d, C*H*₃, δ4.11, q, C*H*), (δ1.46, d, C*H*₃; δ3.73, q, C*H*), (δ3.25, s, N(C*H*₃)₃, δ3.59, t, NC*H*₂, δ4.17, m, NCH₂C*H*₂), (δ1.19, d, C*H*₃, δ2.28, m, *CH*_{A}*H*_{B}, δ2.37, m, CH_{A}*H*_{B}, δ4.12, m, C*H*), (δ2.03, br s, COC*H*₃), and(δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*) are described herein.

Metabolites comprising (or consisting of): (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ1.32, d, C*H*₃, δ4.11, q, C*H*), (δ1.46, d, C*H*₃; δ3.73, q, C*H*), (δ3.25, s, N(C*H*₃)₃, δ3.59, t, NC*H₂*, δ4.17, m, NCH₂C*H*₂), (δ1.19, d, C*H*₃, δ2.28, m, C*H*_{A}HB, δ2.37, m, CH_{A}*H*_{B}, δ4.12, m, C*H*), (δ2.03, br s, COC*H*₃), and(δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*. δ3.49, dd, C(3)*H*, δ3.86, d, C(6)*H*₂, δ4.63, d, C(1)*H*) are described herein.

It is described herein that, wherein said metabolites comprise two or more metabolites selected from: (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ1.32, d, C*H*₃, δ4.11, q, C*H*), (δ1.46, d, C*H*₃; δ3.73, q, C*H*), (δ3.25, s, N(C*H*₃)₃, δ3.59, t, NC*H*₂, δ4.17, m, NCH₂C*H*₂), (δ1.19, d, C*H*₃, δ2.28, m, C*H*_{A}H_{B}, δ2.37, m, CH_{A}*H*_{B}, δ4.12, m, C*H*), (δ2.03, br s, COC*H*₃), and(δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)*H*, δ3.40, dd, C(4)*H*, δ3.47, dd, C(5)*H*, δ3.49, dd, C(3)*H*, δ3.86, d, C(6)*H*₂, δ4.63, d, C(1)*H*); the reference standard is a secondary progressive (SP) phase MS standard, and said concentration differences are selected from:
an increase in the concentration of one or more blood metabolites selected from:(δ1.46 d, C*H*₃; δ3.73, q, C*H*), (δ3.25, s, N(C*H*₃)₃, δ3.59, t, NC*H*₂, δ4.17, m, NCH₂C*H*₂), (δ2.03, br s, COC*H*₃), and(δ3.42, dd, C(4)*H*, δ3.44, dd, C(6)*H*_{A}H_{B}*,* δ3.54, dd, C(2)*H*, δ3.73, dd, C(3)*H*, δ3.79, dd, C(6)H_{A}*H*_{B}, δ3.86, m, C(5)*H*, δ5.24, d, C(1)*H*) and/or (δ3.26, dd, C(2)H, δ3.40, dd, C(4)*H*, δ3.47, dd, C(5)*H*, δ3.49, dd, C(3)*H*, δ3.86, d, C(6)*H*₂, δ4.63, d, C(1)*H*); and a decrease in the concentration of:(δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ1.32, d, C*H*₃*,* δ4.11, q, *CH),* and (δ1.19, d, C*H*₃, δ2.28, m, C*H*_{A}H_{B}, δ2.37, m, CH_{A}*H*_{B}, δ4.12, m, *CH).*

Concentration differences comprising (or consisting of) an increase in the concentration of:
(δ1.46, d, C*H*₃; δ3.73, q, C*H*), (δ3.25, s, N(C*H*₃)₃, δ3.59, t, NC*H*₂*,* δ4.17, m, NCH₂C*H*₂), (δ2.03, br s, COC*H*₃), and(δ3.42, dd, C(4)*H*, δ3.44, dd, C(6)*H*_{A}H_{B}, δ3.54, dd, C(2)*H*, δ3.73, dd, *C(3)H,* δ3.79, dd, C(6)H_{A}*H*_{B}, δ3.86, m, *C(5)H,* δ5.24, d, C(1)*H*) and/or (δ3.26, dd, *C(2)H,* δ3.40, dd, C(4)*H*, δ3.47, dd, *C(5)H,* δ3.49, dd, *C(3)H,* δ3.86, d, C(6)*H₂,* δ4.63, d, C(1)*H*); and a decrease in the concentration of: (δ0.90, br, C*H*₃, δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ1.32, d, C*H*₃*,* δ4.11, q, C*H*), and (δ1.19 d, C*H*₃*,* δ2.28, m, C*H*_{A}H_{B}, δ2.37, m, CH_{A}*H*_{B}, δ4.12, m, *CH)* are described herein.

It is described herein that, wherein said metabolites comprise at least two blood metabolites selected from: (δ2.13, m, C*H*₂CHNH₂, δ2.45, m, OCC*H*₂, δ3.77, t, CH₂C*H*NH₂), (δ1.46, d, C*H*₃; δ3.73, q, C*H*), (δ3.42, dd, C(4)*H*, δ3.44, dd, C(6)*H*_{A}H_{B}, δ3.54, dd, C(2)*H*, δ3.73, dd, C(3)*H*, δ3.79, dd, C(6)H_{A}*H*_{B}, δ3.86, m, C(5)*H,* δ5.24, d, C(1)*H*) and/or (δ3.26,
dd, C(2)*H*, δ3.40, dd, C(4)*H*, δ3.47, dd, C(5)*H,* δ3.49, dd, C(3)*H,* δ3.86, d, C(6)*H*₂, δ4.63, d, C(1)*H*), (δ3.25, s, N(C*H*₃)₃, δ3.59, t, NC*H*₂*,* δ4.17, m, NCH₂C*H*₂), (δ1.32, d, C*H*₃*,* δ4.11, q, *CH),* (δ0.90, br, C*H*₃*,* δ1.30, br, C*H*₂CH₂CH₃, δ1.55, br, C*H*₂CH₃, δ2.05, br, C*H*₂CH=, δ2.25, br, C*H*₂COOH, δ5.35, br, CH₂C*H*=), (δ1.19, d, C*H*₃, δ2.28, m, C*H*_{A}H_{B}, δ2.37, m, CH_{A}*H*_{B}, δ4.12, m, C*H*), and (δ2.03, br s, COC*H*₃) (as described above); said concentration differences confirm the presence of relapsing remitting (RR) phase MS.

Wherein the method described herein permits diagnosis of a disease phase of MS (as described above), the sample is derived from a blood sample and the reference standard is derived from blood.

Wherein the method described herein permits diagnosis of a disease phase of MS (as described above), the sample is derived from a serum or plasma sample and the reference standard is derived from serum or plasma.

In one embodiment, the method of the invention further comprises recording the output of at least one step on a data-storage medium. By way of example, the method of the present invention can generate data relating to the test subject, such data being recordable on a data-storage medium (for example, a form of computer memory such as a hard disk, compact disc, floppy disk, or solid state drive). Such data can comprise (or consist of) data relating to the concentration in a sample (from said test subject) of any of said two or more metabolites (as described) above.

Described herein is a data-storage medium comprising data obtained by a method described herein.

A device for use in a method as described above, wherein said device is capable of performing the step of identifying a concentration difference for each of said two or more metabolites in the sample relative to the reference sample is described herein.

### List of figures

**Figure 1**
   (A) A graph showing the clinical scores of the mice with Cr-EAE, CFA scores are not shown due to the fact that the score never deviated from 0. Arrows indicate when the samples were taken from each animal. (B) A graph showing the percentage weight loss of the both the EAE and CFA animals.
**Figure 2**
   (A) ¹H NMR NOESY-presat spectrum of a urine sample from a Day 38 Cr- EAE animal. (B) PLS-DA plot of animal urine samples comparing Cr-EAE animals at Day 38 (black squares) and Cr-EAE animals at Day 28 (grey squares). (C) PLS-DA plot of animal urine samples comparing Cr-EAE animals at Day 38 (black squares) and naive animals (grey triangles). (D) PLS-DA plot of animal urine samples comparing Cr-EAE animals at Day 38 (black squares) and CFA animals at Day 38 (grey circles) (E) Table showing the q² values of all the animal models. (F) Table to show the q² values of Cr-EAE and CFA animals at different time-points compared to the naive group.
**Figure 3**
   Overlaid ¹H NMR spectra (1D spectra recorded with solvent presaturation and CPMG background suppression) of serum from relapse remitting (blue) and secondary progressive (red) patients with key metabolites identified. Enlarged sections of spectra: (i) Anomeric region of α-glucose; (ii) Region containing the resonances due to sugars; (ii) Region containing fatty acid and methyl resonances. Key: 1: Water (*H*2O), 2: Glucose resonances 3: β-Glucose anomeric protons 4: Fatty acid =CH-C*H2*-, 5: N-acetyl species (C*H*₃), putative assignment based on published data (Fan, 1996; Wishart, *et al.,* 2009), 6: Alanine (C*H*3), 7: Fatty acid peak (C*H*3) 8: Fatty acid peak (-C*H2*- chain), 9: Lactate *(CH3),* 10: Lactate (C*H*) 11: α-Glucose anomeric protons, 12: Unsaturated fatty acid (CH2C*H*=C*H*CH2).
**Figure 4**
   (A) ¹H NMR NOESY-presat spectrum of a Cr-EAE animal plasma sample at Day 38. B) PLS-DA plot of animal plasma samples comparing Cr-EAE animals at Day 38 (black squares) and Cr-EAE animals at Day 28 (grey squares). (C) PLS-DA plot of animal plasma samples comparing Cr-EAE animals at Day 38 (black squares) and naive animals (grey triangles). (D) PLS-DA plot of animal plasma samples comparing Cr-EAE animals at Day 38 (black squares) and CFA animals at Day 38 (grey circles) (E) Table showing the q² values of all the animal models. (F) Table to show the q² values of Cr-EAE and CFA animals at different time-points compared to the naive group.
**Figure 5**
   (A) Sample ¹H NOESY-presat NMR spectrum of human urine. (B) PLS-DA plot of human serum samples comparing Relapse Remitting patients (grey diamonds) and Secondary Progressive patients (black circles). (C) PLS-DA plot of human urine samples comparing Secondary Progressive patients (grey triangles) and a control group (black circles). (D) PLS-DA plot of human serum samples comparing Secondary Progressive patients (grey triangles) and a control group (black circles). (E) Table to show the q² values of the human urine samples from all the models. (F) Table to show the q² values of the human serum samples from all the models.
**Figure 6**
   A box and whisker plot showing the take one out analysis of the RR v SP model. Stars indicate p < 0.0001 difference between the means of the two groups, box shows standard deviation, mid-line shows mean of two groups. Whiskers show the upper and lower limits of the data.
**Figure 7**
   **Supplementary Information 1:** Sample model validation plot, observed q² value outlined square and generated q² values black squares. The generated q² values are generated within Simca, in order to consider the plot to be valid all generated q² values must be lower than the observed q² value for the model. The y axis shows the correlation of the generated values when compared to the observed value.
**Figure 8**
   **Supplementary Information 2:** A sample plasma COSY spectra from a day 38 Cr- EAE animal showing highlighted correlations which assisted the metabolite identification. Key 1: Fatty acid, 2: Glutamate, 3: Taurine, 4: Citrate, 5: Alanine, 6: Lactate, 7: Region containing glucose correlations, 8: β-glucose, 9: α-glucose.
**Figure 9**
   **Supplementary Information 3:** Table of all chemical shifts of the metabolites identified in the results section as given in the literature. Key: δ - chemical shift, M - multiplicity, s - singlet, d - doublet, t - triplet, q - quartet, dd - double doublet, ddd - double double doublet, m - multiplet, br - broad resonance.
**Figure 10**
   **Supplementary Information 4:** NMR spiking experiments. (A) ¹H NMR spectrum of potential metabolites in order to confirm the identity of certain metabolites with a singlet resonance. (B) Baseline NOESY ¹H NMR of animal urine (C) Addition of ureidopropionic acid (D) Addition of TMAO (E) Addition of phosphocholine (F) Addition of TMA
**Figure 11**
   **Supplementary information 5:** Table showing both up and down regulated metabolites which cause separation within the animal urine models.
**Figure 12**
   **Supplementary Information 6:** (A) Table showing the positive and negative metabolites which cause the separation in the Cr-EAE plasma models when compared to animals at day 10. (B) Table showing the positive and negative metabolites which cause the separation in the Cr-EAE plasma models when compared to animals at day 14. (C) Table showing the positive and negative metabolites which cause the separation in the Cr-EAE plasma models when compared to animals at day 17. (D) Table showing the positive and negative metabolites which cause the separation in the Cr-EAE plasma models when compared to animals at day 21.
**Figure 13**
   **Supplementary Information 7:** (A) Table showing the positive and negative plasma metabolites which cause the separation between Cr-EAE and naive animals at different time-points. (B) Table showing the positive and negative plasma metabolites which cause the separation between Cr-EAE and CFA animals at different time-points. (C) Table showing the positive and negative plasma metabolites which cause the separation between CFA and naive animals at different time points.
**Figure 14**
   **Supplementary Information 8:** (A) Table showing the first round of positive and negative metabolites which caused the separation in the human urine model. (B) Table showing the first round of positive and negative metabolites which caused the separation in the human serum models. (C) Table showing the second round of positive and negative metabolites which caused the separation in the human serum models.
**Figure 15**
   **Supplementary Information 9:** Schematic to show the metabolite changes over time in the animal models. (A) Schematic showing metabolite changes within the Cr- EAE animals with respect to naive animals. (B) Schematic showing metabolite changes within the Cr-EAE animals with respect to CFA animals. Shaded bars represent period of active disease.
**Figure B1**
   Human analysis: (A) PLS-DA plot of human serum samples comparing Relapse Remitting patients (open grey triangles) and a control group (filled black squares). (B) PLS-DA plot of human serum samples comparing Relapse Remitting patients (filled black diamonds) and Secondary Progressive patients (open grey triangles). (C) Table to show the q2 values of the models from human serum samples. n.p. = not predictive (i.e. q2 < 0).
**Figure B2**
   ROC curves + contingency tables: (A, B, C) 2x2 contingency tables for human model prediction results summarising correct and incorrect identifications. In each case, Fisher's exact 2-tailed p-values are less than 0.05 (Control vs. RRMS, p = 0.0013; Control vs. SPMS, p =5.1x10-5; SPMS vs. RRMS, p = 0.017). (D) Receiver operator characteristic curves constructed for each of the three human models validated with a testing set of patients. Control vs. SPMS is black squares and black line. Control vs. RRMS is grey triangles and grey line. SPMS vs. RRMS is open circles and black line.
**Figure B3**
   Tables of patient information. A. Data for whole population comparison. * = p<0.05 with respect to RRMS. *** = p<0.001 with respect to RRMS. ††† = p<0.001 with respect to PPMS, RRMS and SPMS. ‡‡‡ = p<0.001 with respect to RRMS. B. Comparisons within and between sets of patients. i.e. comparisons were made between groups within each set (e.g. RRMS vs. SPMS within Set A) as well as between the same group from each set (e.g. RRMS Set A vs. RRMS Set B). Where no significance is listed, p-values were > 0.05. a = p<0.05 with respect to SPMS from Set B and Set C. b = p<0.001 with respect to RRMS from Set A. c = p<0.01 with respect to RRMS from Set A. d = p<0.001 with respect to control from Set A. e = p<0.05 with respect to control from Set A and p<0.01 with respect to SPMS from Set A. f = p<0.05 with respect to control from Set B. g = p<0.001 with respect to control from Set B. h = p<0.001 with respect to control from Set C. Data are medians (min-max).
**Figure B4**
   Sample model validation plot. Observed q2 value is outlined square and generated q2 values are black squares. The generated q2 values are derived by randomising samples to different groups in multiple combinations and calculating the q2 value for all tested permutations. In order to consider the plot to be valid >95% of generated q2 values must be lower than the observed q2 value for the model. The y axis shows the correlation of the generated values when compared to the observed value.
**Figure B5**
   A sample plasma COSY spectra from an SPMS patient showing highlighted correlations which assisted the metabolite identification. Key 1: Fatty Acids, 2: Glutamate, 3: Taurine, 4: Citrate, 5: Alanine, 6: Lactate, 7: Region containing glucose correlations, 8: β-glucose, 9: α-glucose.
**Figure B6**
   Table of chemical shifts of metabolites. Key: d - chemical shift, M - multiplicity, A - Assignment of proton, s - singlet, d - doublet, t - triplet, q - quartet, dd - double doublet, ddd - double double doublet, m - multiplet, br - broad resonance.
**Figure B7**
   Urine NMR spiking experiments. 1H NMR spectrum of potential metabolites in order to confirm the identity of metabolites with a singlet resonance. (A, blue) Baseline 1H NMR NOESY of animal urine. (B, red) Addition of TMAO. (C, Green) Addition of phosphocholine.
**Figure B8**
   Table to show the q2 values of the models from serum samples along with key metabolites identified.
**Figure B9**
   Summary statistics and characteristics for ROC curves produced with the testing set of independent samples (Set C) for the modelling. SE area is standard error of the area. P-values are calculated with the null hypothesis that the area is 0.5 (i.e. random assignment of patients to classes).
**Figure B10**
   (A) PLS-DA plot of urine samples comparing Secondary Progressive patients (open grey triangles) and a control group (filled black circles). (B) Table to show the q2 values of the models from human urine samples along with key metabolites identified.

### Examples

The following materials and methods were used in the examples described below.

### Materials and Methods

### Animal Preparation

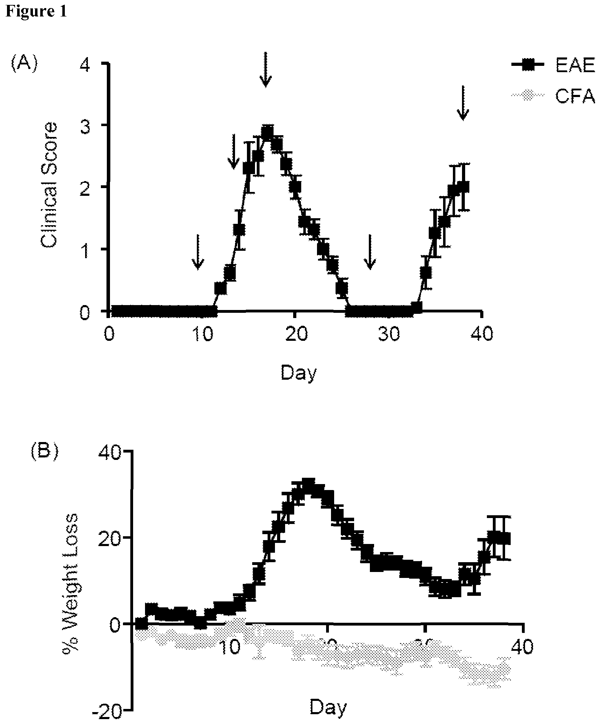

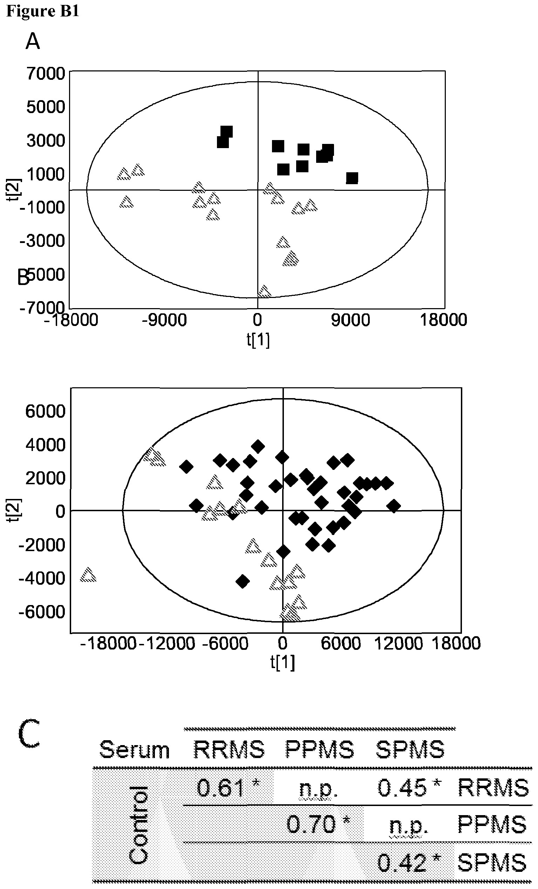

Urine and plasma samples were obtained from a mouse model of chronic relapsing experimental autoimmune encephalomyelitis (Cr-EAE), a more clinically-relevant model for MS than the commonly used monophasic disease models. Adult Biozzi ABH mice were fed on a diet of jelly and high protein mash in the cage for a period of one week prior to Cr-EAE induction and throughout the disease time course. Cr-EAE was induced as described by Baker et al. (J Neuroimmunol. 1990; 28(3): 261-70). Briefly, each animal was injected subcutaneously on day 0 and day 7 with mouse spinal cord homogenate in incomplete Freund's adjuvant supplemented with non-viable desiccated, *M. tuberculosis* and *M. butyricum.* A group of control animals was injected with the supplemented incomplete Freund's adjuvant (CFA), but omitting the spinal cord homogenate. Animals were weighed daily and assessed for clinical signs (Figure 1).

Blood and urine samples were taken at days 10, 14, 17, 28 and 38 after Cr-EAE induction. Blood was collected by cardiac puncture using a heparin-coated syringe and placed immediately into a blood tube containing dipotassium ethylenediaminetetraacetic acid (EDTA) on ice (Teklab, UK). Blood samples were subsequently centrifuged (14000 rpm, 5 min) at 4 °C, after which the top layer of plasma was removed and stored at -80 °C. Urine samples were either collected directly from the bladder after sacrificing the animal with CO₂ or by placing a 96-well plate underneath the animal during sacrifice.

### Human Samples

Blood serum (n=58) and urine samples (n=84) were obtained from MS patients (n=58) under Ethical approval (C03.054, Metabolic profiling of urine in MS) with cohorts of relapsing remitting (RR) (urine n = 22; serum n = 19), primary progressive (urine n = 32 ; serum n = 13) and secondary progressive (urine n = 30; serum n = 26) MS patients. Control samples were collected from age and sex matched volunteers (urine n = 28; serum n = 16).

### NMR Spectroscopy

Urine or blood serum/plasma samples were defrosted overnight in a cold room prior to the NMR experiments and then blood samples were further centrifuged (100000 g, 30 mins). The urine and plasma samples (100 µl EAE or 150 µl MS) were placed in a 5 mm NMR tube and diluted to a final volume of 600 µl with phosphate buffer (0.2 M Na₂HPO₄/0.04 NaH₂PO₄, pH 7.4, 0.1% sodium azide, sodium chloride 0.8%) in D₂O containing 1mM TSP (3-trimethylsilyl-1-[2,2,3,3,-²H₄] propionate) as an internal standard. ¹H NMR spectra were acquired from each sample using a 16.4T NMR (700 MHz ¹H) system (Bruker Avance III equipped with a ¹H TCI cryoprobe). For all samples a 1D NOESY presaturation sequence, with solvent pre-saturation during the relaxation delay (2s) and mixing time (10ms), was used. For plasma samples alone a CPMG sequence was also used with solvent presaturation and a T₂ filter (total duration 40ms; 20 loops of 2ms spin-echoes) for suppression of lipid resonances.

Two dimensional 1H NMR spectra were acquired from a sample within each group to assist with metabolite identification. The 2D Correlation Spectroscopy (COSY) spectra were acquired on the same spectrometer as the 1D NMR spectra.

The COSY spectra were acquired with 1.5s solvent presaturation, a spectral width of 10 ppm (7002 Hz), and 16 or 32 transients per t₁ increment for 256 increments. All NMR experiments were acquired at 293K.

The 1D ¹H plasma spectra were sub-divided into 0.02 ppm regions (δ = midpoint of integral region) and integrated, reducing each spectrum to 435 independent variables between 0.20 - 4.30 and 5.00 - 9.60 ppm. The integration was carried out using a macro within ACD 1D NMR Processor release 12 (Advanced Chemical Development, UK) The 1D ¹H urine spectra were sub-divided into 0.02 ppm integral regions and integrated, reducing the spectrum to 385 independent variables between 0.20-4.30 and 6.00-9.60 ppm. In all spectra, the region between 4.30 and 5.00 ppm was omitted due to spectrum distortion arising from the water suppression at 4.7 ppm. In the case of the urine spectra the region between 5.00 and 6.00 ppm was also excluded to avoid the broad resonance arising from urea. These sections represent highly variable regions of the spectrum. Subsequently, statistical pattern recognition was applied to the spectra to differentiate between samples obtained from different disease states in either the EAE model or from the MS patients.

All statistical modelling was carried out using SIMCA P+ 12.0 (Umetrics, Sweden). All data was scaled using Pareto variance in order to suppress the noise in the data. In order to determine how predictive the models were the q² value for each model was used as a guide to the validity of the PLS-DA model. A q² value above 0.4 was considered statistically significant (Waterman, *et al.,* 2010) and, therefore, tested further. The validate function within Simca P+ was used to generate 100 models where the samples were placed into random classes. The q² values of these models 10 were then calculated and compared to the actual q² value of the genuine model. The model was considered valid if all the predictive q² values were lower than the real q² value (Supplementary Information 1).

The models were further validated by removal of individual points within the PLS models. The models were then rebuilt and the omitted sample was then reintroduced in order to confirm that it fell within the requisite group (so-called "take one out" analysis). In order for a perfect classification the samples scored 1 if they were a member of the required group or 0 if they were not a member. An arbitrary cut-off value of 0.5 was applied to samples which did not perfectly fit the model (Brindle, *et al.,* 2002; Gavaghan, *et al.,* 2000). The difference between the groups was then tested with an unpaired t test to investigate the differences between the groups. If the models failed these tests they were classed as non-significant (ns), however, if they passed all the tests they were considered to be predictive models.

In order to identify the metabolites the loadings were examined by use of an s-plot, and the peaks were identified using a combination of COSY spectroscopy (Supplementary Information 2), literature values and reference to the human metabolome database (Supplementary Information 3) (Fan, 1996; Wishart, *et al.,* 2007; Wishart, *et al.,* 2009). Further confirmation of the metabolites was achieved by examining the *J*-coupling (spin-spin interactions between neighbouring hydrogens) of the resonances within the spectra.

In order to confirm the identity of the singlet peaks which give no correlations within the 2D COSY spectra the predicted metabolite was added ('spiked') into the NMR tube as an authentic reference sample (Supplementary information 4 A).

### Example 1

### Cr-EAE: Animal Model

All Cr-EAE animals followed the typical disease course reported previously by Baker *et al.* 1990; the animals exhibited significant weight loss and increased clinical score, which peaked at day 17, and was followed by full remission such that clinical score had returned to baseline values in all animals by day 26 (Figure 1). Subsequently, the animals entered a relapse phase, around day 32, with a second significant increase in clinical score and loss of weight. During peak disease Cr-EAE animals were hand-fed to reduce dehydration and weight loss. Injection of CFA alone (CFA group) did not cause any overt clinical signs or significant weight loss (Figure 1).

### Example 2

### Cr-EAE: Urine analysis

Spectra obtained by high resolution ¹H NMR spectroscopy were of sufficient resolution to allow identification of over 30 metabolites. PLS analysis of the urine spectra from the naive animals at different time-points returned a model with a low q² (0.10), indicating that it was not predictive. Consequently, all naive data were combined as a single group thereafter and analysis performed using a single control (naive) group.

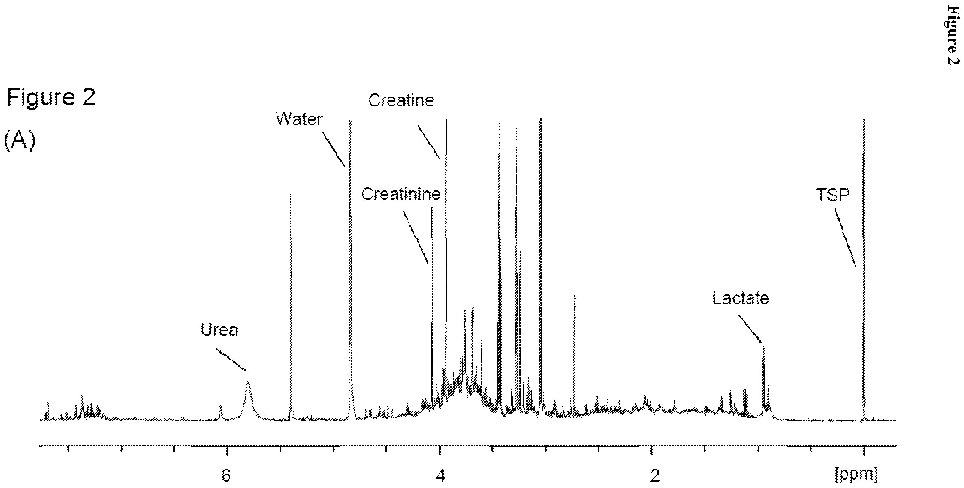

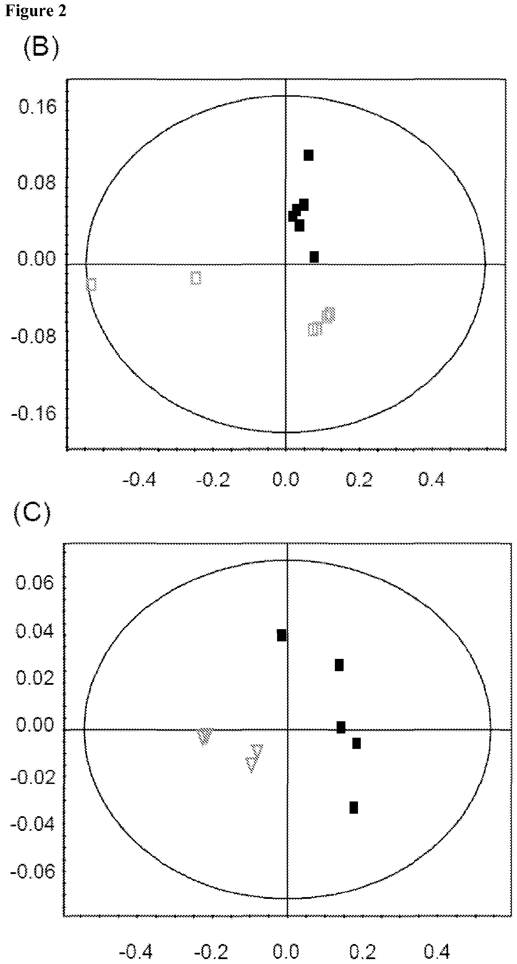

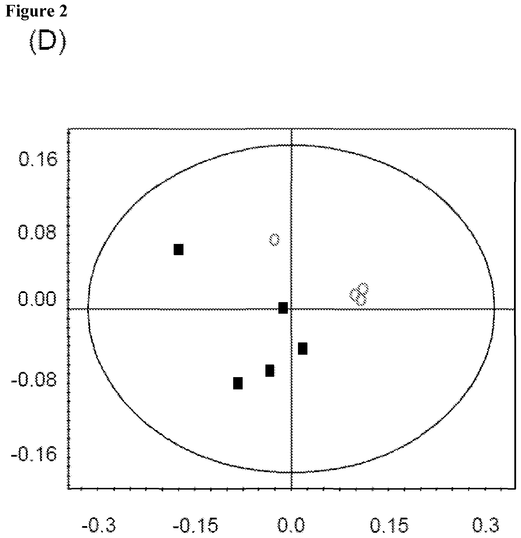

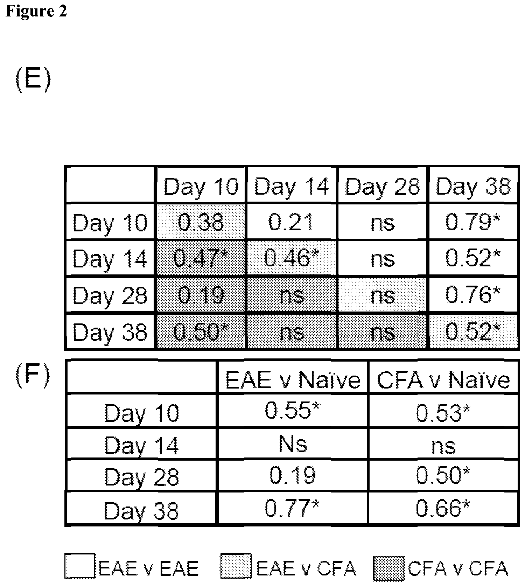

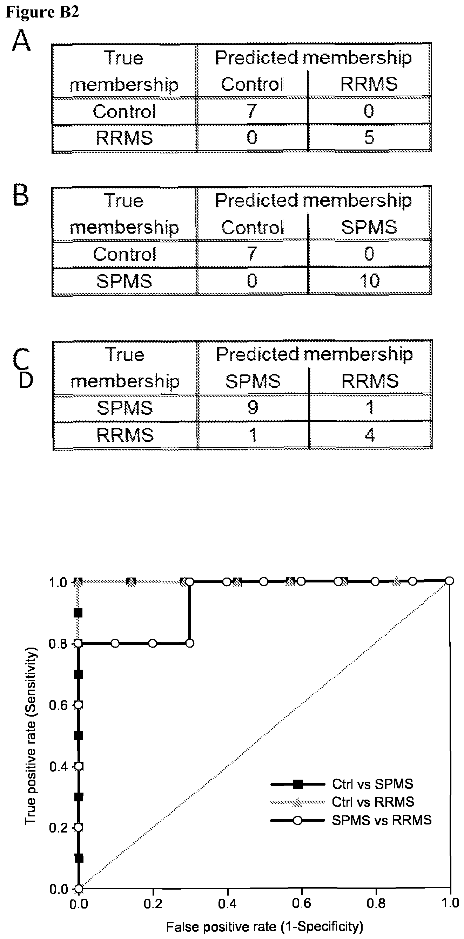

Visually, there appeared to be separation of the different disease groups, as illustrated in Figure 2B, C and D. However, although many of the urine models returned a positive q² value indicating differences both within the Cr-EAE animals at different time points and between the Cr-EAE and control (CFA or naive) animals, only 12 models reached statistical significance (q² ≥ 0.4; Figure 2,Table E and F). At day 10 it was possible to separate both the Cr-EAE and the CFA animals from the naive animals (q² = 0.55 and 0.53, respectively; (Figure 2, Table E and F), although, whilst strongly positive the separation between Cr-EAE and CFA animals at this time point did not quite reach statistical significance (q² = 0.38). However, a significant separation was found between the Cr-EAE and CFA animals at day 14 with a q² value of 0.46 (Figure 2, Table E). Cr-EAE animals at day 38 showed a positive separation from all other Cr-EAE time points and also from the CFA and naive controls (Figure 2, Table E and F). No data were obtained from the animals at day 17 as the volume of urine collected was not adequate.

CFA animals at day 10 showed a positive separation from both days 14 and 38 (q² = 0.47* and 0.5, respectively; Figure 2, Table E), and at day 28 it was possible to separate the CFA animals from the naive cohort (q² 0.5) (Figure 2, Table F).

The loadings from the statistically significant models generated for the disease time course identified several key metabolites that were responsible for the separations between groups (see supplementary 3 for full literature assignments). Here δ_{x-y} is defined as the chemical shift of the centre of the region identified by the pattern recognition within SIMCA. In particular, citrate (δ_{x-y} 2.50, 2.65), creatine (δ_{x-y} 3.03), taurine (δ_{x-y} 3.25, 3.40), an unassigned set of resonances (δ_{x-y} 3.13, two singlets in close proximity to each other), trimethylamine-N-oxide (TMAO; δ_{x-y} 3.26), trimethylamine (TMA; δ_{x-y} 2.89) and ureidopropionic acid (δ_{x-y} 2.37) all varied significantly over the disease time course. The identity of these last three metabolites was confirmed by spiking experiments (Supplementary Information 4). With the exception of ureidopropionic acid, the same metabolites were also identified when comparing Cr-EAE animals with the two control groups (CFA and naive). In addition, 14 phosphocholine (δ_{x-y} 3.23), also confirmed by spiking (Supplementary Information 4E), was an important contributor to the model separating Cr-EAE and CFA animals at day 38. The direction of change in each metabolite varied over the disease time course. For example, at days 10 and 14, which are in the pre-clinical and early onset phases of disease, the levels of citrate were lower in Cr-EAE animals than in control animals, whilst during relapse at day 38 they were elevated compared to the CFA controls. In contrast, the levels of the TMAO resonance peaked in Cr-EAE animals during the remission phase just prior to relapse (day 28), but then remained elevated at day 38 (peak relapse) when compared to the pre-clinical and early symptomatic time-points (day 10 and day 14; Supplementary Information 5).

### Example 3

### Cr-EAE: Plasma analysis

Two different NMR sequences were used to obtain spectra from the plasma samples; (i) the NOESY presaturation sequence used for the urines and (ii) a CPMG sequence with a T₂ lipid suppression filter. For some samples phase modulation was evident in the CPMG spectra and, therefore, the NOESY presaturation spectra were used in all subsequent analysis.

As for the urine, PLS analysis of the plasma spectra from the naive animals at different time-points returned a model with a low q² (0.176), indicating that it was not predictive. Consequently, all naive data were combined as a single group thereafter and analysis performed using a single control (naive) group.

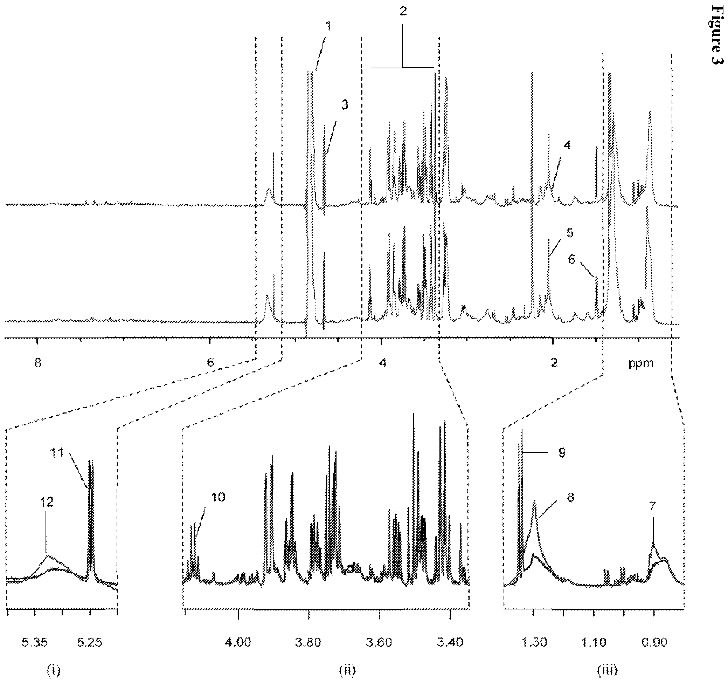

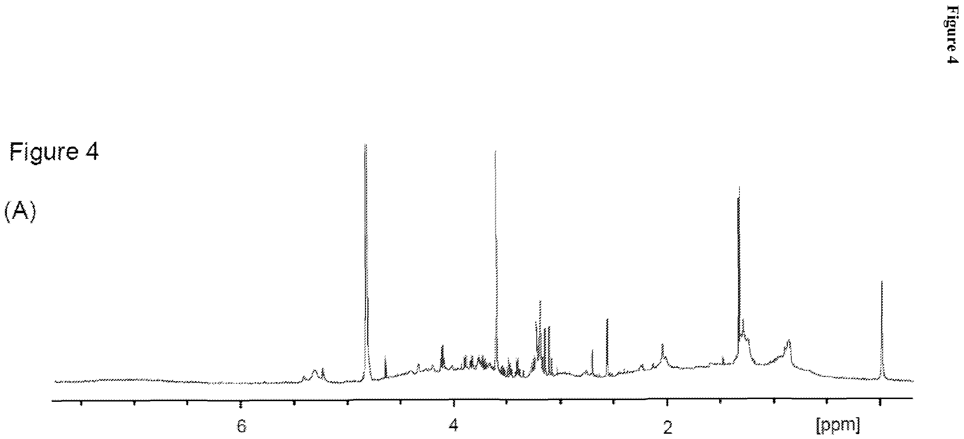

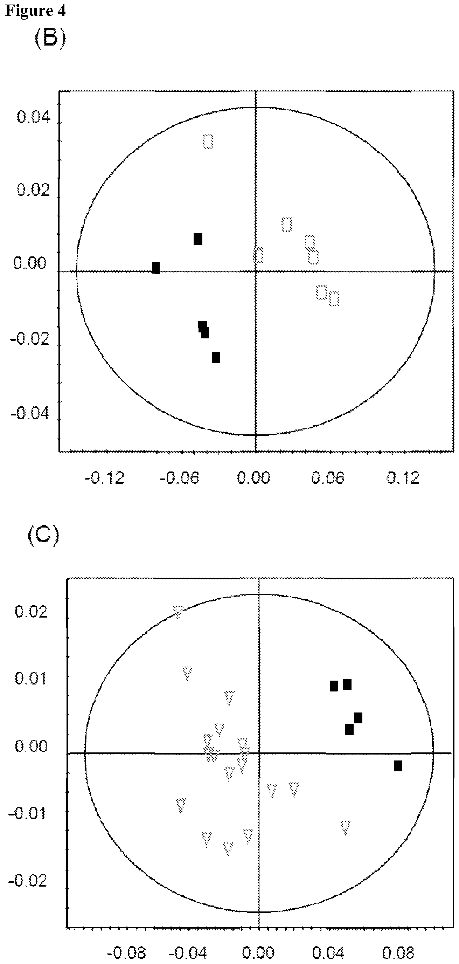

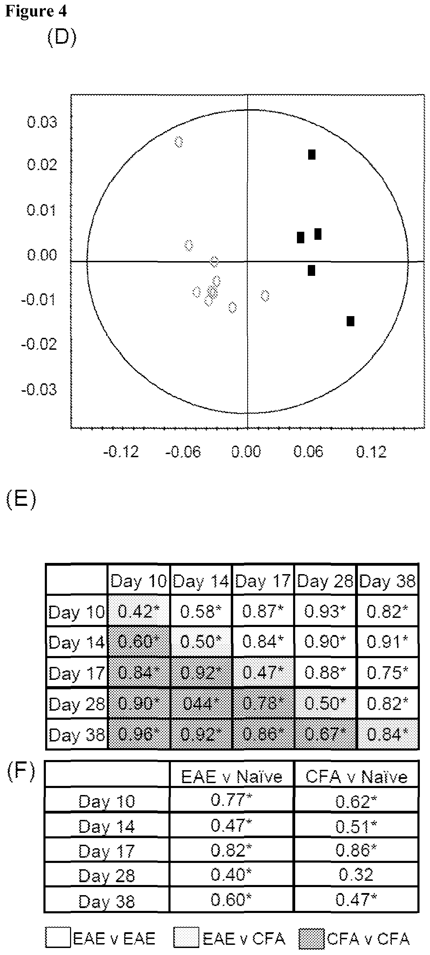

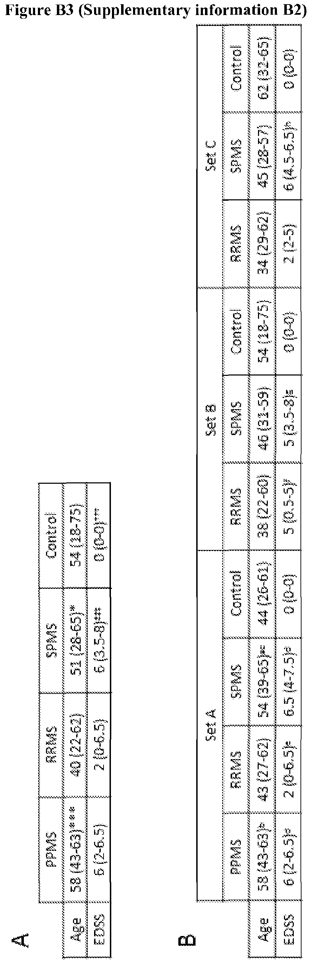

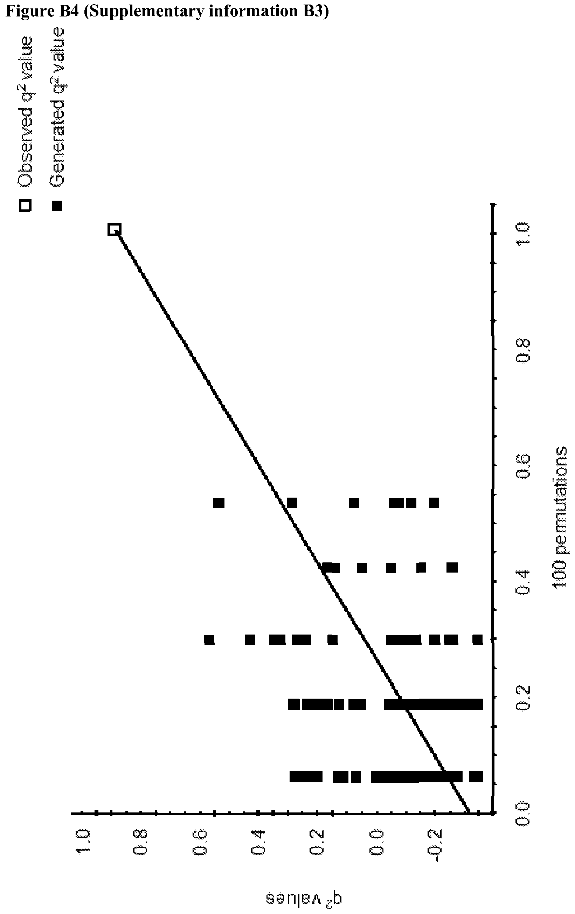

Although the spectra from all groups superficially look similar upon initial overlay (Figure 3) the multi-variant statistical analysis of the data sets showed predictive separations between the Cr-EAE groups at all time-points during the disease course and also when comparing Cr-EAE to both CFA and naive animals (Figure 4, Table E and F). It was also possible to generate predictive models based on the spectra from the plasma of the CFA animals at all-time points during the disease course, with the exception of day 14 *vs.* day 28 (Figure 4, Table E).
On examination of the loadings within the PLS models for the Cr-EAE time course several key metabolites were identified as generating the positive separations. The initial positive models generated by comparing the different time-points for the Cr-EAE animals showed variations in n-butyrate (δ_{x-y} 0.88, 1.58) and trimethylamine N-oxide (TMAO) (δ_{x-y} 3.44). Following exclusion of these metabolites from the original spectra it was possible to generate further positive models with significant q² values. Across all of the time point comparisons the only models that were not statistically significant were the comparisons between Cr-EAE and CFA at days 17 and 28, i.e. during Cr-EAE remission (Supplementary Information 6). Taurine (δ_{x-y} 3.25) and lactate (δ_{x-y} 1.32) were identified as metabolites that played a key role in generating the second round of positive models.

Key metabolites from the original models separating Cr-EAE animals from the control groups (CFA or naive) were also identified. The metabolites that showed greatest variation in these models were: fatty acid (δ_{x-y} 0.88, 1.58, 2.03), citrate (δ_{x-y} 2.5, 2.65), lactate (δ_{x-y} 1.32, 4.11), glucose (δ_{x-y} 3.25) and phosphocholine (δ_{x-y} 3.23). As above, removal of these metabolites from the original spectra generated further positive models with significant q2 values. The metabolites that generated the positive q2 values in the second round of models were: taurine (δ_{x-y} 3.25, 3.43) and valine (δ_{x-y} 0.99, 1.04) (Supplementary Information 7; A and B). When the data from 16 CFA animals were compared with the naive cohort it was again possible to separate the animals at all time-points except day 28 (Supplementary Information 7; C).

All of the metabolites above showed both positive and negative changes compared to baseline over the disease time course (Supplementary Information 7; A and B). In the case of taurine, a consistent pattern was found when comparing the Cr-EAE animals to both the CFA and the naive animals, such that during peak disease, days 10 and 38, taurine increased in the Cr-EAE animal group compared with both control groups. Similarly, glucose and phosphocholine metabolites showed an increase relative to naive animals during periods of active disease (e.g. days 14 and 38), whilst phosphocholine metabolites fell during remission (day 28). However, the relative concentration of glucose was lower at both days 10 and 28 in Cr-EAE animals than in CFA animals, but higher at day 38.

In contrast to the increases observed above, the concentration of fatty acid in Cr- EAE animals was reduced during active disease, days 10, 14 and 38, relative to naive animals, but elevated during remission (day 28). At these time-points the levels of fatty acid in the CFA animals were even lower than in the Cr-EAE animals, indicating a greater effect of CFA alone than Cr-EAE on these metabolites.

No differences in TMAO were evident between Cr-EAE and naive animals. However, TMAO was lower over the early disease time course (days 10, 14 and 17) in Cr-EAE animals than CFA animals, and higher at day 38, again indicating differential effects of CFA and Cr-EAE.

### Example 4

### Human MS: Urine analysis

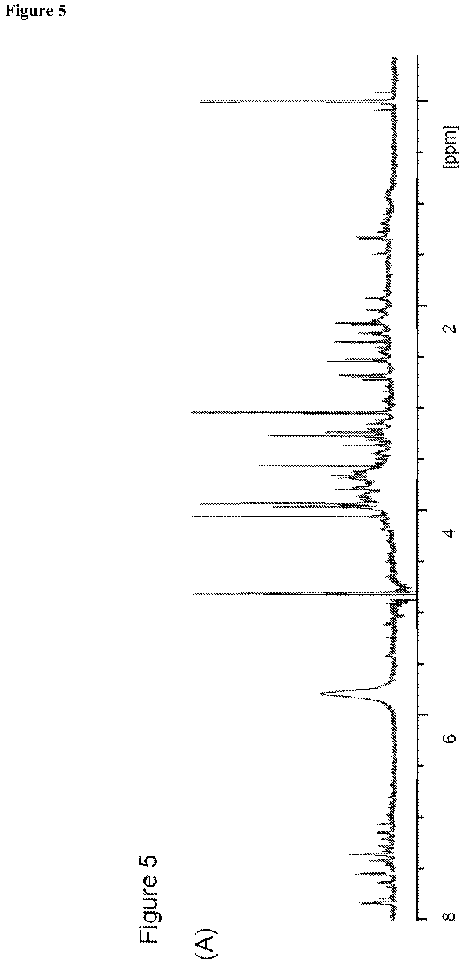

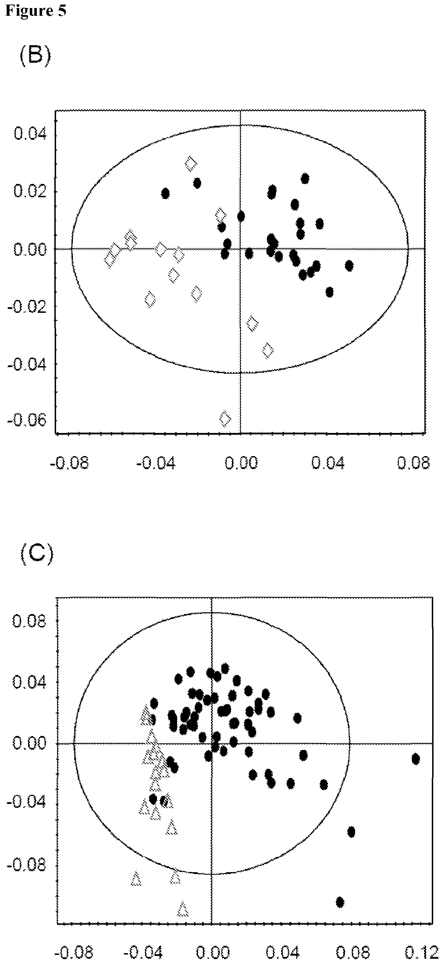

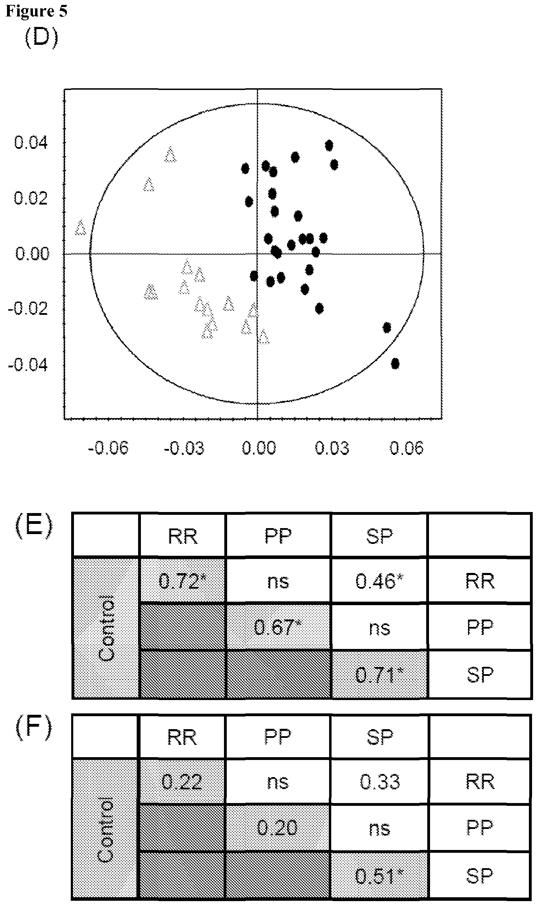

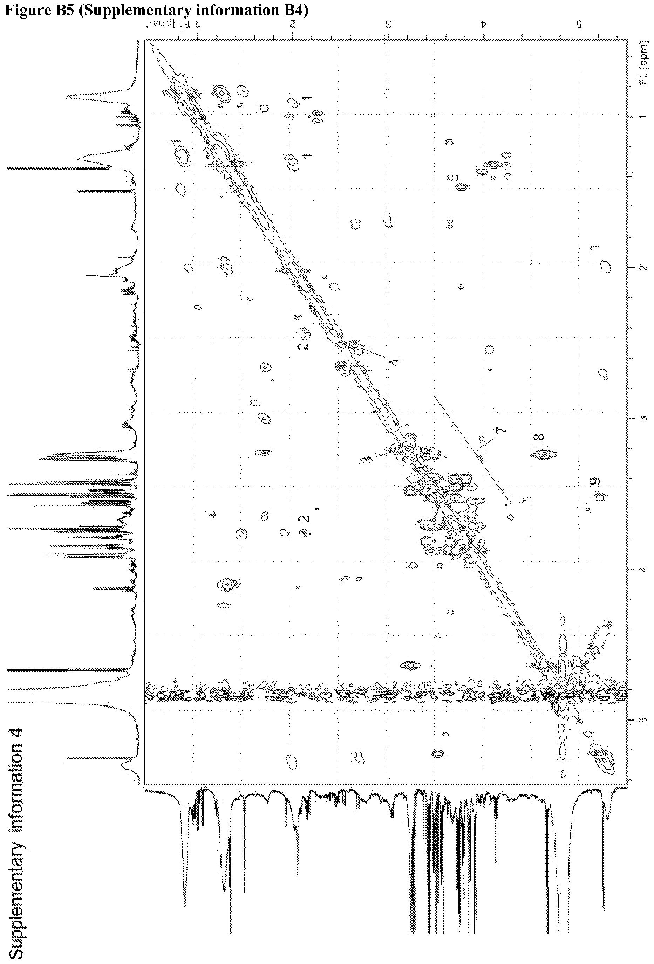

Initially, all of the MS patients were modelled against the control group. However, although positive (q² = 0.11), this model did not reach significance. When the different stages of disease were considered separately against controls, all of the PLS models again returned positive q² values indicating differences between each of these groups (RR, PP, SP) and the healthy control groups. However, only the SP *vs*. control model reached defined significance (q² = 0.51, take-one-out analysis *p* < 0.05; Figure 5E). On examination of the loadings, citrate and TMAO were increased in the SP group compared to the control group (Supplementary Information 8; Table A). Relative levels of lactate and creatinine (δ_{x-y} 4.06) were reduced in the SP group compared to control. When comparing RR and PP against the healthy control group the q² values were only 0.22 and 0.20, respectively, despite showing a visual separation of the groups. With respect to separations between the disease groups, comparison of the RR and SP groups showed a clear separation visually, but the q² value of 0.33 did not quite reach significance. No significant differences were found between the PP group and either the SP or RR groups (Figure 5; Table E).

### Human MS: Serum analysis

The same two NMR sequences as used for the animal samples were run on the human serum samples. However, unlike the spectra obtained from the animal samples, no phase modulation was evident in the spectra obtained from the human samples. Since there were no differences between the two types of spectra, the CPMG spectra were used in all subsequent analysis. The high resolution ¹H NMR spectroscopy furnished spectra of sufficient resolution to identify over 30 metabolites (Figure 5 A).

Once again the whole cohort of MS patients was compared to the healthy control group. This model showed a significant separation between the two groups with a q² value of 0.40; take-one-out analysis *p* < 0.05. Subsequent to this, the different disease states were considered individually. All of the individual comparisons between the MS patient groups (RR, PP, SP) and the healthy control group (Figure 5B, C and D) proved to be highly significant against the defined criteria of q2 > 0.4 (q2 = 0.73, 0.64, 0.70, respectively). On examination of the spectra from the control group, three of the control samples contained an unknown contaminant (δ_{x-y} 3.66, 3.73). These buckets were excluded from the analysis and new models were generated, which all reached the defined significance (q² = 0.72, 0.67, 0.71, respectively). Interestingly, when comparing each of the individual stages of MS (RR, PP and SP) against the controls, three metabolites, glucose (δ_{x-y} 3.25, 3.75, 3.91), phosphocholine (δ_{x-y} 3.23; confirmed by spiking) and an unidentified metabolite (singlet δ_{x-y} 3.37), were consistent across the groups (Supplementary Information 8; Table B). Glucose and phosphocholine were reduced in all MS patients relative to healthy controls, whilst the unknown singlet was higher in all MS groups. For the RR group, the relative concentrations of L-glutamine (δ_{x-y} 2.15) and L-alanine (δ_{x-y}1.47) were also higher than in healthy controls. When the loadings for the SP *vs*. control model were examined, no further metabolites were elevated in the SP group compared to the control group, but fatty acid were reduced. In contrast, fatty acid were elevated in the PP group compared to controls, as was a resonance assigned to *N*-acetyl species (δ_{x-y} 2.03).

Following the initial analysis, the metabolites responsible in each group for causing separations were removed from the spectra of that particular group and a second 19 round of positive models were generated (Supplementary Information 8; Table C). When comparing the RR and healthy control groups, lactate was found to be reduced in the RR patients. Lactate was also the only other metabolite that varied in the SP *vs*. control model, and, similarly, was reduced in the SP patients. When comparing the PP and control groups, L-glutamine was found to be elevated in the PP samples, whilst an unknown singlet (δ_{x-y} 2.65) was reduced.

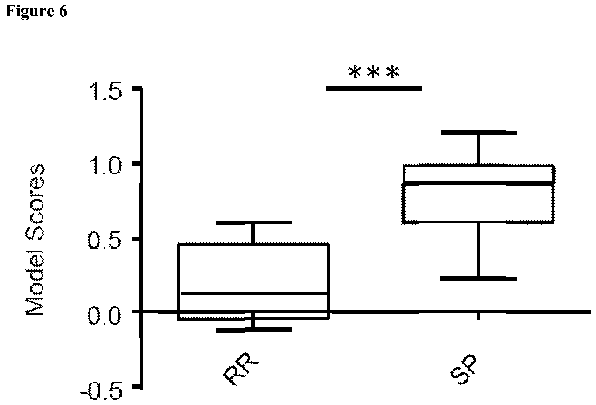

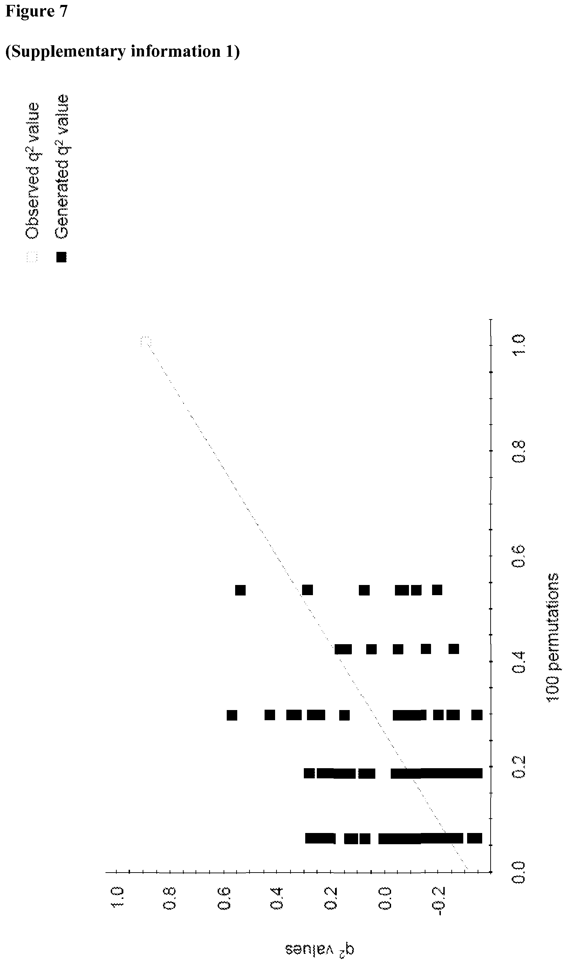

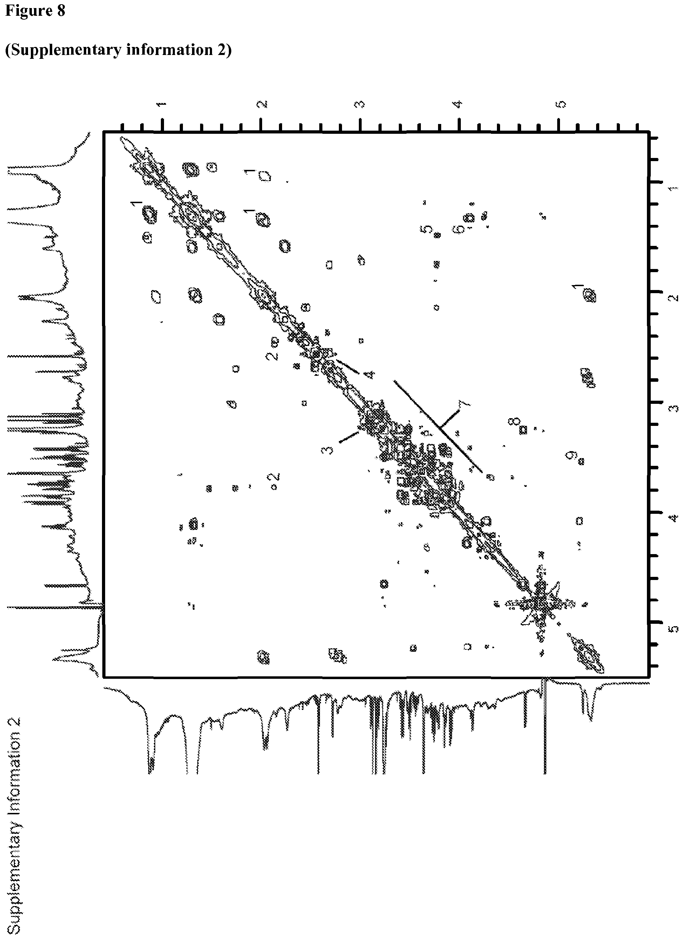

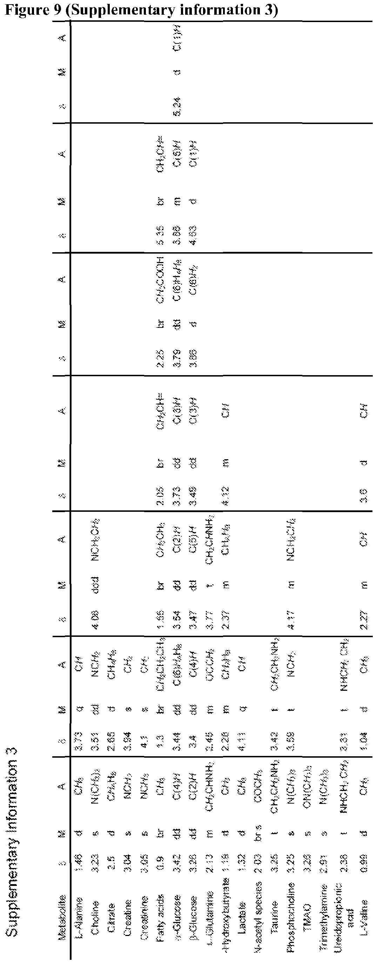

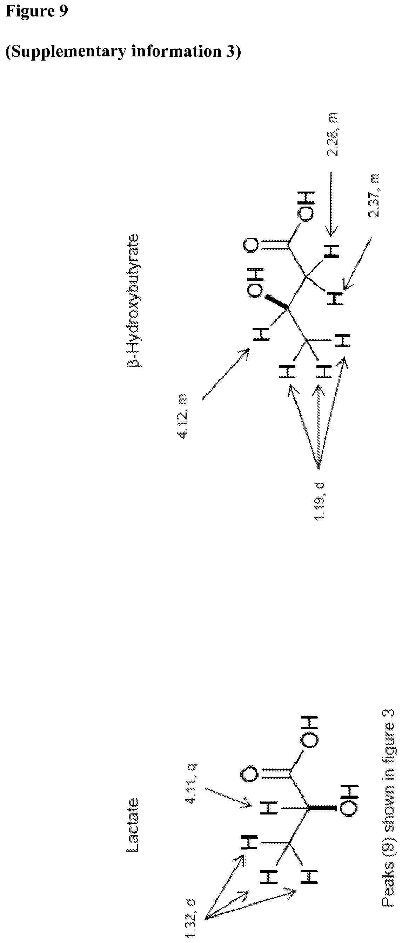

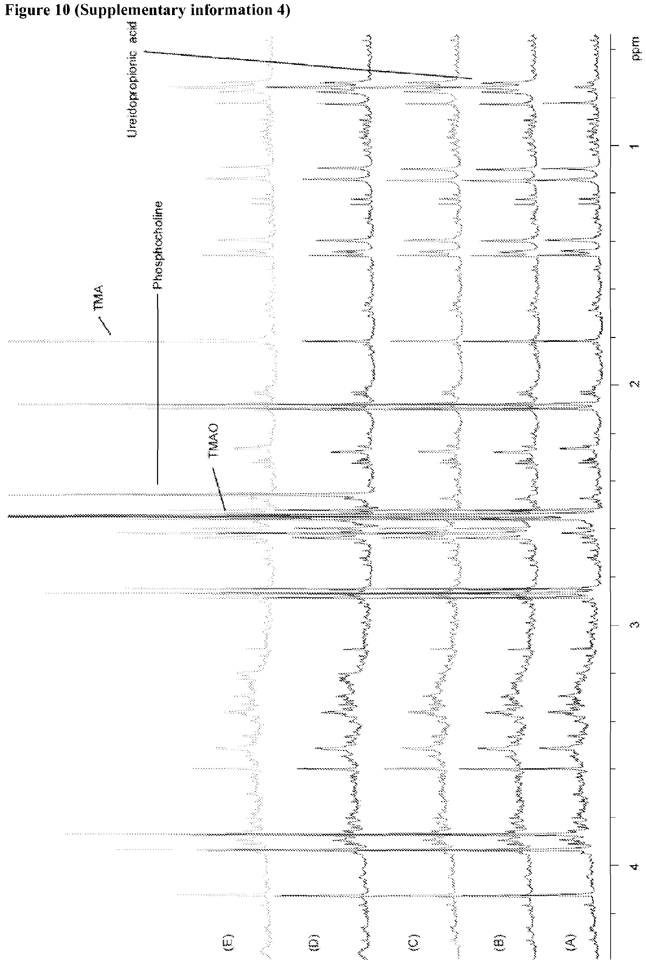

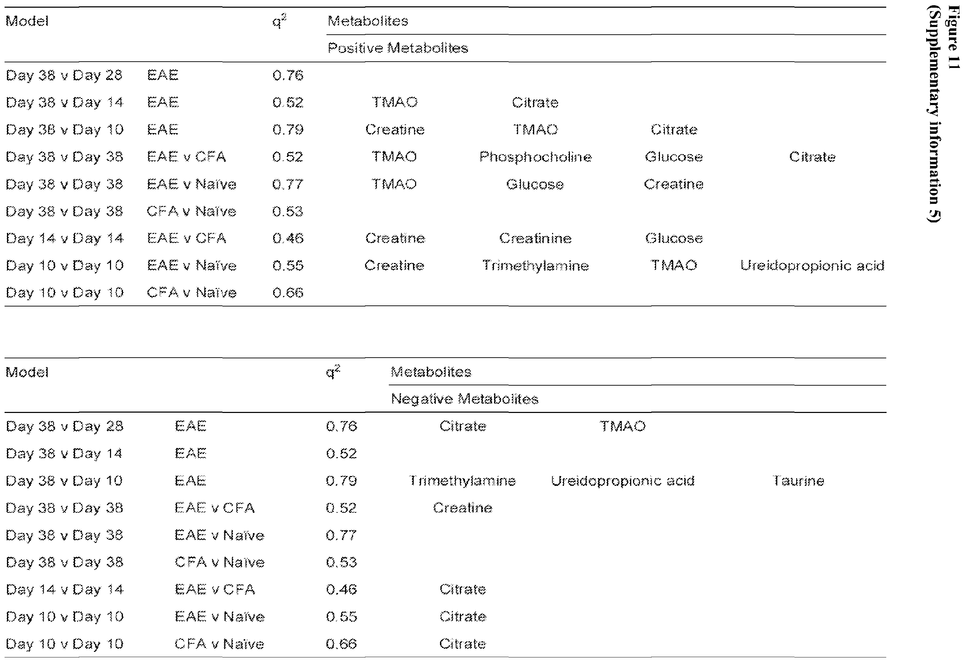

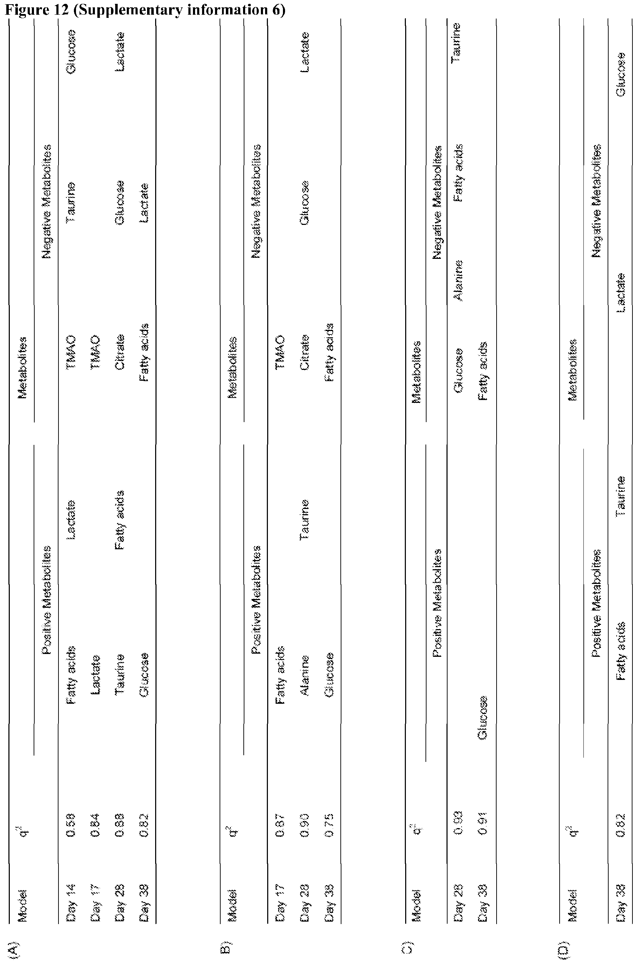

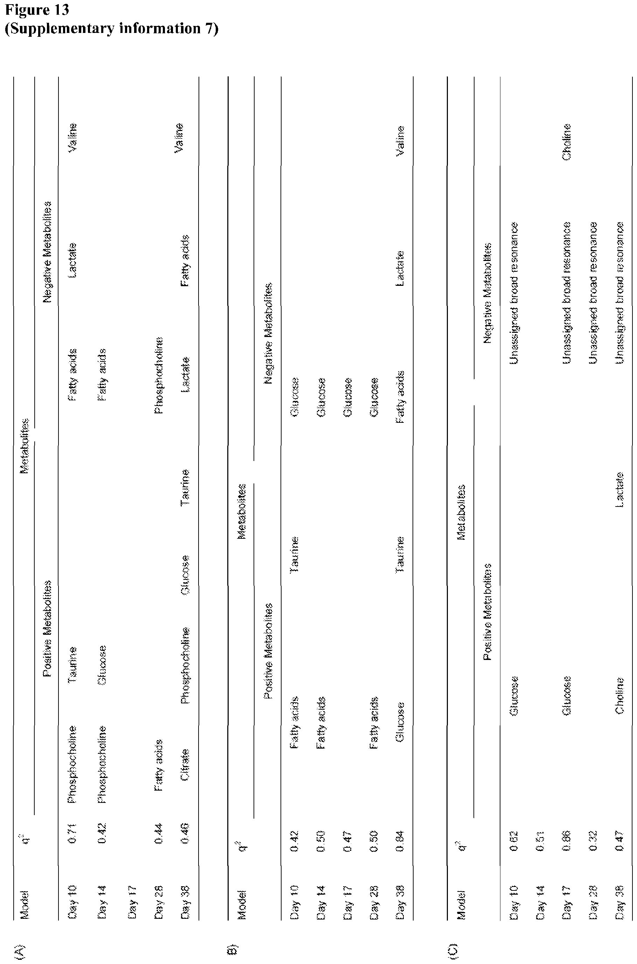

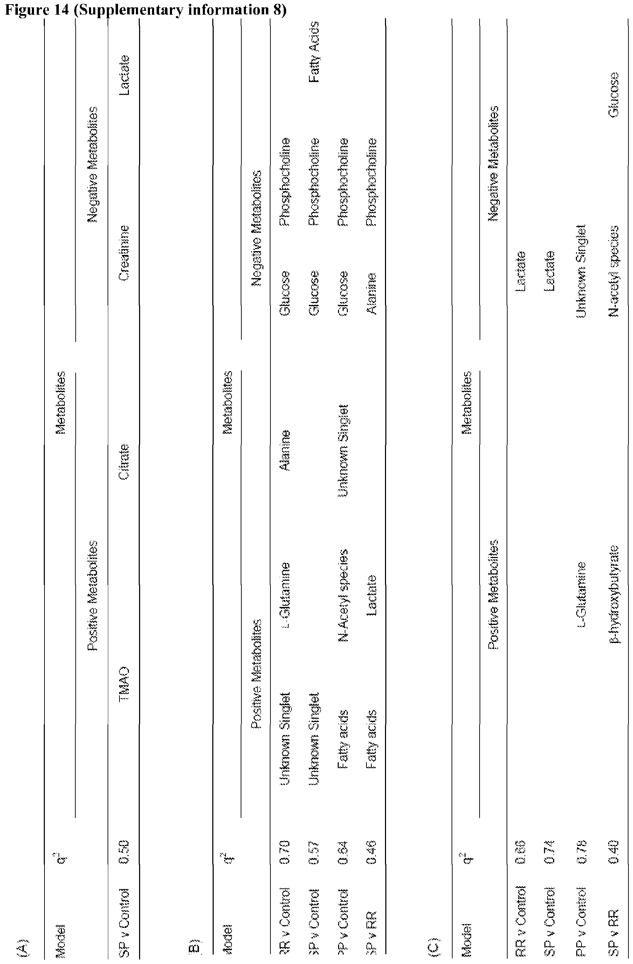

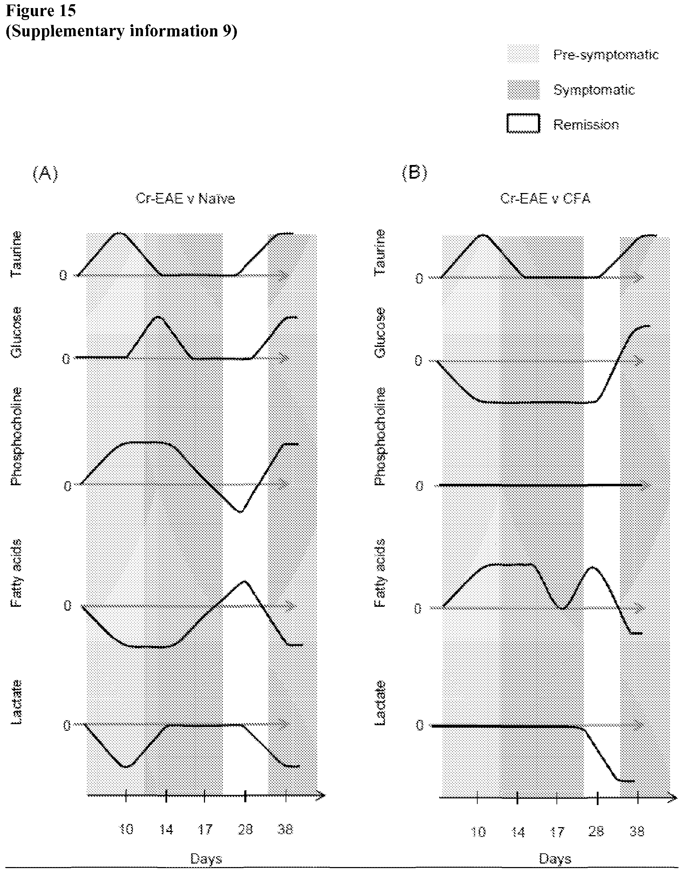

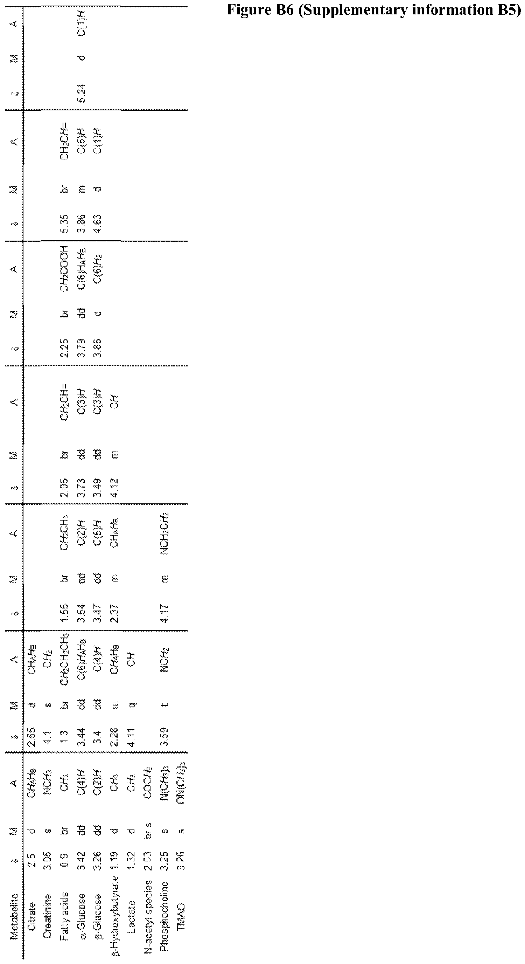

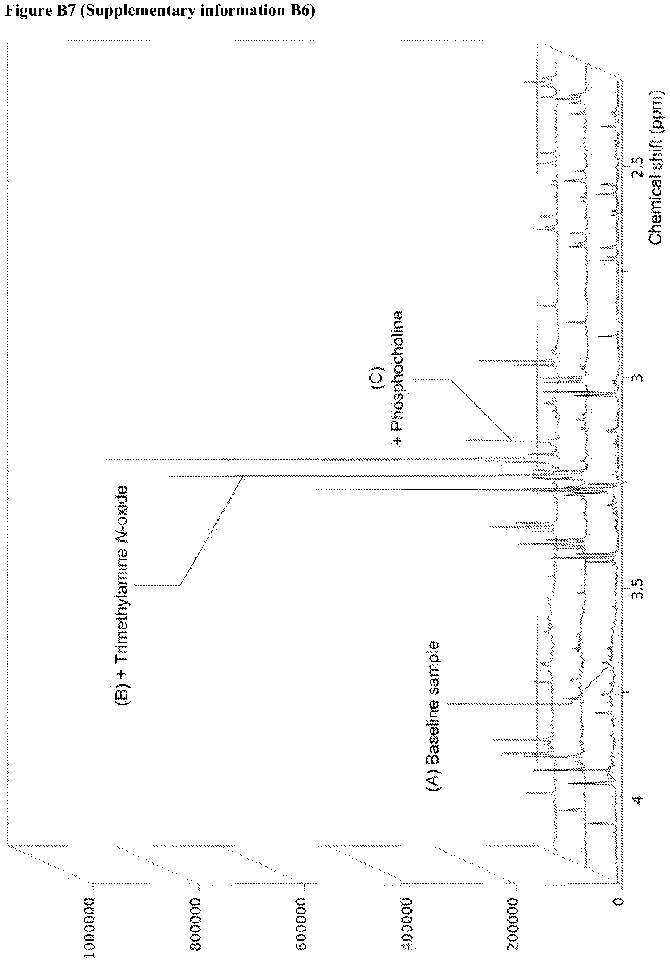

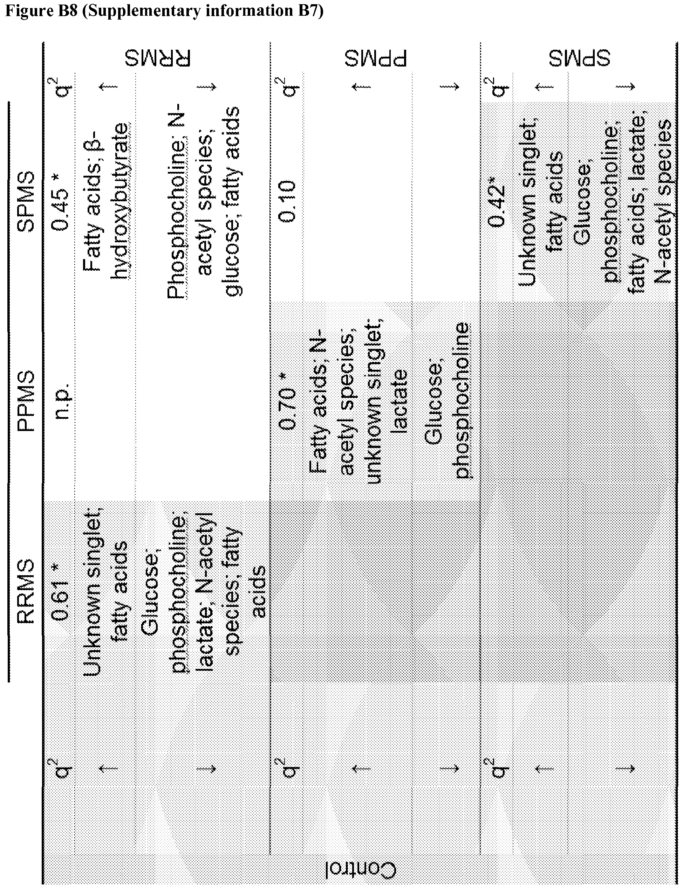

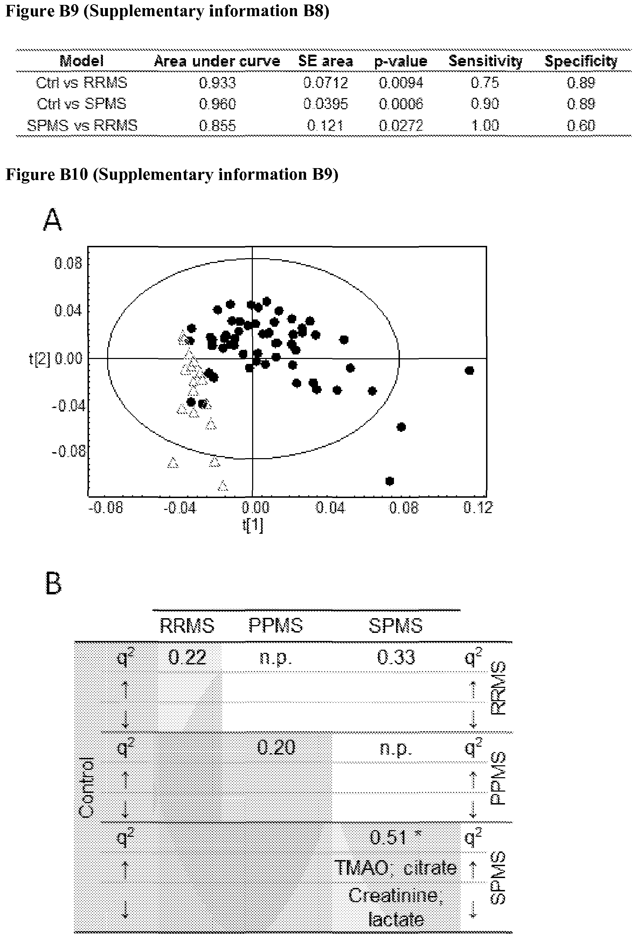

Comparison of the RR and SP groups generated a significant model, with a q² value of 0.46. Subsequent take-one-out analysis generated a box and whisker plot which showed a statistically significant difference between the two groups with a p value < 0.0001 (Figure 6), further confirming this to be a predictive model. Although the model comparing the PP and RR groups appeared to show two clear groups, a few overlapping outliers in each group resulted in a negative q2 value (-0.19). Consequently, this model could not reliably differentiate between PP and RR MS (Figure 4; Table F). Comparison of the PP and SP groups returned a positive, but non-significant, q² value (0.1), and overlap of the groups was evident.

In the case of the RR *vs*. SP model, after the first round of analysis fatty acid (δ_{x-y} 0.88, 1.58 1.3) and lactate (δ_{x-y} 1.32) were found to be higher in the SP group, whilst alanine (δ_{x-y} 3.73) and phosphocholine (δ_{x-y} 3.23) were found to be lower in the SP group than the RR group (Supplementary Information 8; Table B). Removal of these regions from the spectra generated a further positive model (q² = 0.40), reflecting higher levels of beta-hydroxybutyrate (δ_{x-y} 2.43) and lower levels of glucose, and a broad singlet like resonance which was tentatively assigned as an *N*-acetyl species, 20 (previously assigned as *N-acetyl glycoprotein* (Griffin, *et al.*, 2004)), in the SP group (Supplementary Information 8; Table C).

### Example 5

### SUBJECTS/MATERIALS AND METHODS

### Human Samples

Samples from three cohorts of MS patients as well as age- and sex-matched control volunteers were obtained with ethical approval (UK NHS C03.054, Metabolic profiling of urine in MS and 08/H0604/155): Set A: urine n=22, 30 and 28, serum n=15, 38 and 10; Set B: serum n=6, 10 and 7; Set C: serum n=5, 10 and 7 in RRMS, SPMS and control volunteers respectively. Set A urine and serum samples were also collected for primary progressive (PP) MS patients (n=32 and 13 respectively). Serum samples were collected by centrifugation (3000xg, 10mins) after allowing blood to clot for 30 min at room temperature in Vacutainers containing clot activator and gel (BD, UK). Urine samples were collected mid-stream in pots without additives. Serum and urine were aliquoted and stored at -80°C. Clinical details of MS patients are given in Supplementary Information B2. Human patient samples were excluded from analysis if patients were found to suffer from another disease (e.g. diabetes), if the spectral quality was poor or distorted in a gross fashion or if the prepared sample was found to have a lipid content greater than 2xSD from the mean of the group.

### NMR Spectroscopy

Samples were defrosted at 4°C prior to NMR. Blood samples were further centrifuged (100,000xg, 30min, 4°C). Urine and plasma (100µl EAE or 150µl MS) were diluted in a 5mm NMR tube to a final volume of 600µL with 0.24M sodium phosphate buffer (pH 7.4, 0.1% NaN₃, 0.8% NaCl) in D₂O containing 1mM TSP (3-trimethylsilyl-1-[2,2,3,3,-²H₄] propionate) as an internal standard. ¹H NMR spectra were acquired from each sample using a 16.4T NMR (700MHz ¹H) system (Bruker Avance III equipped with a ¹H TCI cryoprobe). For all samples a 1D NOESY pre-saturation sequence, with solvent presaturation during the relaxation delay (2s) and mixing time (10ms), was used. For blood samples alone a CPMG sequence was also used with solvent pre-saturation (2s) and a T₂ filter (total duration 40ms; 20 loops of 2ms spin-echoes) for suppression of lipid resonances. All 1D spectra were automatically baseline corrected using a 0^{th} order polynomial (Topspin 3.0). Two-dimensional Correlation Spectroscopy (COSY) ¹H NMR spectra were acquired from a sample within each group to assist with metabolite identification. The COSY spectra were acquired with 1.5s solvent presaturation, a spectral width of 10ppm (7002Hz), and 16 or 32 transients per t₁ increment for 256 increments. All NMR spectra were acquired at 293K.

### Data analysis

Non-linear peak alignment¹⁶ was performed on each 1D ¹H spectra which were then sub-divided into 0.02ppm regions (δ = midpoint of integral region) and integrated between 0.20-9.60ppm using a custom MATLAB script. In all spectra, the highly variable region between 4.30-5.00ppm arising due to water suppression was excluded. In urine spectra, the variable urea resonance between 5.00-6.00ppm was also excluded. Thus spectra were reduced to 435 (blood) or 385 (urine) independent variables. Data were scaled using the Pareto variance to suppress noise and PLS/DA was applied. Integral buckets containing gross contaminants (e.g. organic solvent, EDTA) were excluded from analysis.

### Statistical methods

For each comparison, a PLS-DA model was derived which best explained the differences between the variables for the groups being studied (SIMCA P+ 12.0, Umetrics, Sweden). To determine the potential predictive nature of the models, the q² value was calculated. q² is derived from a step-wise cross-validation of the model whereby a model generated with a subset of samples removed is used to predict group membership of the missing samples. A value of q²>0 means the model is predictive. It is generally held that q²>0.4 is the threshold for significance for biological modelling¹⁷.

In addition, model validation was carried out using a pseudo-Monte Carlo method where 100 models were built using random group assignments. Only models where the genuine q² was higher than 95% of the randomly generated q² values were considered significant (Supplementary Information B3).

In order to identify the metabolites underlying group separations, the variable loadings of each model were examined and the relevant resonances identified using a combination of COSY spectroscopy (Supplementary Information B4), literature values and reference to the human metabolome database (see Supplementary Information B5 for full NMR assignments)¹⁸⁻²⁰. To confirm identity of singlet peaks, predicted metabolites were 'spiked' into samples as authentic references (Supplementary Information B6).

To validate the models generated additional sample sets (Sets B and C) were used. PPMS patients were not included owing to lack of samples. Set B was used to build predictive models and Set C was used as a testing and validation set i.e. Set C were blinded and the models built using Set B were used to predict group membership for each sample in Set C. The results are expressed as a 2x2 contingency table for each model. Fisher's exact statistic was calculated in each case. Receiver operator characteristic (ROC) curves were constructed for each model and the area under the curve determined. Model membership probability thresholds derived from the model-building set were used to determine sensitivity and specificity in the prediction set (Set C).

### RESULTS

### Initial MS sample set (Set A)

We began with analysis of serum samples from MS patients in Set A. A model built using the CPMG spectra and separating all MS patients from the control cohort was predictive (q²=0.41). Furthermore, the comparisons between individual MS patient and control groups were significant (q²=0.70, 0.61 and 0.42 for control vs. PPMS, RRMS and SPMS respectively; Figure B1A and C). All models were successfully validated using the cross-validation method described above. Interestingly, when comparing each of the individual stages of MS against the controls, three metabolites were consistent across the groups. Glucose (δ_{x-y} 3.25, 3.75, 3.91) and phosphocholine (δ_{x-y} 3.23; confirmed by spiking, (Supplementary Information B5 and B6) were reduced whilst an unidentified metabolite (singlet δ_{x-y} 3.34) was increased in all MS patients relative to healthy controls. Further examination of both the RR vs. control and SP vs. control model loadings showed lactate (δ_{x-y} 1.32), a broad singlet-like resonance tentatively assigned to *N*-acetyl species (δ_{x-y} 2.03, previously assigned as *N*-acetyl glycoproteins¹⁴) and some fatty acids were decreased whilst other fatty acids were elevated. Examination of the PP vs. control model loadings showed that lactate, the *N*-acetyl species and some fatty acids were also increased. Detailed metabolite findings are shown in Supplementary Information B7.

When making comparisons between MS groups, the model comparing the RR and SP groups revealed a significant separation (q² = 0.45; Figure B1B and C). Examination of the loadings revealed that fatty acids (δ_{x-y} 0.88, 1.30, 5.35), phosphocholine (δ_{x-y} 3.23), the *N*-acetyl species, (δ_{x-y} 2.03) and glucose (δ_{x-y} 3.25, 3.75, 3.91) were decreased in SPMS with respect to RRMS whilst other fatty acids and β-hydroxybutyrate (δ_{x-y} 1.19) were increased. For other intra-MS comparisons, the model comparing the PP and RR groups appeared to show two clear groups, however a few overlapping outliers in each group resulted in a non-predictive q² value (-0.11). Consequently, this model could not reliably differentiate between PP and RR MS. Similarly, comparison of the PP and SP groups returned a non-predictive q² value (-0.02), and overlap of the groups was evident.

The urine analysis produced models which were not as strong as those built from the serum samples. Models constructed comparing control volunteer urine to each of the MS groups were all predictive but only control *vs*. SPMS reached the defined significance of q²>0.4 (Control *vs*. RRMS q² = 0.22, *vs*. PPMS q² = 0.20, *vs.* SPMS q² = 0.51). For the intra-MS urine comparisons, both PPMS *vs*. RRMS and PPMS *vs*. SPMS models proved to be non-predictive whilst SPMS *vs*. RRMS proved predictive but non-significant (q² = 0.33; Supplementary Information B1 and B9).

### Independent MS sample set analysis (Sets B and C)

To validate the models generated from sample Set A, two independent sets of serum samples were used, Sets B and C. Samples from Set B were used in the same manner as the samples from Set A and three new models were generated: Control_{B} *vs*. RRMS_{B}, Control_{B} *vs*. SPMS_{B} and RRMS_{B} *vs*. SPMS_{B}. In each case, the models from Set B were predictive and were all validated in the same manner as the models from Set A. As previously, the q² values were greater than 0.4 for each model: Control_{B} *vs*. RRMS_{B}, q² = 0.62; Control_{B} *vs*. SPMS_{B} , q² = 0.77; and RRMS_{B} *vs*. SPMS_{B}, q² = 0.48. Moreover, upon examination of the loadings, the same metabolites were found to be responsible for the separations observed between groups in this secondary dataset with the exception of glucose, which was not as important in separating SPMS from RRMS patients in the secondary data set. This is likely to be owing to variation in sample handling technique in the clinic e.g. time of clinic or duration since last meal.

Each new model generated from Set B was tested for its predictive ability using Set C, the second independent data set which had not been used in building any models. All Set B models were both sensitive and specific at predicting group membership for patients: RRMS *vs*. SPMS model, sensitivity = 0.9 and specificity = 0.8; RRMS and SPMS *vs*. control models, sensitivity and specificity = 1.0. Contingency tables showing correct and incorrect classifications for each model are given in Figure B3A to C. In each case, two-tailed p-values calculated using Fisher's exact test were less than 0.05 (control *vs*. RRMS, p = 0.0012; control *vs*. SPMS, p = 5.1x10⁻⁵; SPMS *vs*. RRMS, p = 0.017). ROC plots for the models (Figure B3D) yielded areas under the curve (AUC) significantly greater than 0.5, indicating good predictive power (control *vs*. RRMS AUC = 1.00, p = 0.0045; control *vs*. SPMS AUC = 1.00, p = 6.4x10⁻⁴; SPMS *vs*. RRMS AUC = 0.94, p = 0.007), see Supplementary Information B8.

### References

Brindle JT, Nicholson JK, Schofield PM, Grainger DJ, Holmes E. Application of chemometrics to 1H NMR spectroscopic data to investigate a relationship between human serum metabolic profiles and hypertension. Analyst. 2002; 128(1): 32-6.
Fan WMT. Metabolite profiling by one- and two-dimensional NMR analysis of complex mixtures. Progress in Nuclear Magnetic Resonance Spectroscopy. 1996; 28: 161-219.
Gavaghan CL, Holmes E, Lenz E, Wilson ID, Nicholson JK. An NMR-based metabonomic approach to investigate the biochemical consequences of genetic strain differences: application to the C57BL10J and Alpk: ApfCD mouse. FEBS letters. 2000; 484(3): 169-74.
Griffin JL, Anthony DC, Campbell SJ, Gauldie J, Pitossi F, Styles P, et al. Study of cytokine induced neuropathology by high resolution proton NMR spectroscopy of rat urine. FEBS Letters. 2004; 568(1-3): 49-54.
Waterman C, Currie R, Cottrell L, Dow J, Wright J, Waterfield C, et al. An integrated functional genomic study of acute phenobarbital exposure in the rat. BMC genomics. 2010; 11(1): 9.
Whitaker JN, McKeehan A, Freeman DW. Monoclonal and polyclonal antibody responses to the myelin basic protein epitope present in human urine. J Neuroimmunol. 1994; 52(1): 53-60. 35
Whitaker JN, Kachelhofer RD, Bradley EL, Burgard S, Layton BA, Reder AT, et al. Urinary myelin basic protein-like material as a correlate of the progression of multiple sclerosis. Ann Neurol. 1995; 38(4): 625-32.
Wishart DS, Tzur D, Knox C, Eisner R, Guo AC, Young N, et al. HMDB: the Human Metabolome Database. Nucleic Acids Research. 2007; 35(suppl 1): D521-D6.
Wishart DS, Knox C, Guo AC, Eisner R, Young N, Gautam B, et al. HMDB: a knowledgebase for the human metabolome. Nucleic Acids Research. 2009; 37(suppl 1): D603.

## Claims

1. An *in vitro* method for diagnosing Multiple Sclerosis (MS) in a human test subject, comprising:
a.
(i) determining the relative concentrations of three or more metabolites in a sample from said subject, wherein said three or more metabolites are selected from:
blood metabolites, wherein said blood metabolites comprise: phosphocholine, lactate, N-acetyl species, fatty acid, glucose, and L-glutamine;
(ii) comparing the relative concentrations of said three or more metabolites in the sample with the relative concentrations of the same metabolites in at least one reference standard from a subject that does not have MS;
and
(iii) identifying a concentration difference for each of said three or more metabolites in the sample relative to the reference standard, wherein said concentration difference is selected from: an increase in the concentration of one or more blood metabolites selected from: fatty acid, N-acetyl species, and lactate; and
a decrease in the concentration of the blood metabolites glucose and phosphocholine; or
wherein said concentration difference is selected from:
an increase in the concentration of one or more blood metabolites selected from: fatty acid, N-acetyl species, and L-glutamine; and
a decrease in the concentration of the blood metabolites glucose, and phosphocholine;
wherein said concentration differences correlate with the presence of MS;
wherein said N-acetyl species is N-acetyl aspartate and/or N-acetyl glycoprotein; and/or
wherein the fatty acid has a resonance value of δ0.90, br, C*H₃*, δ1.30, br, *CH₂CH₂CH₃,*δ1.55*,* br, *CH₂CH₃,* δ 2.05, br, C*H₂*C*H*=*,* δ 2.25 br, C*H₂*C*OOH,* δ5.35, br, C*H₂*C*H*= relative to a methyl resonance of 3-trimethylsilyl-1-propionate (TSP) defined at 0.00 ppm as determinable by ¹H-NMR;
and/or
b.
(i) determining the relative concentrations of two or more metabolites in a sample from said subject, wherein said two or more metabolites are selected from: urine metabolites, wherein said urine metabolites comprise: citrate, creatinine, lactate and trimethylamine N-oxide (TMAO);
(ii) comparing the relative concentrations of said two or more metabolites in the sample with the relative concentrations of the same metabolites in at least one reference standard from a subject that does not have MS;
and
(iii) identifying a concentration difference for each of said two or more metabolites in the sample relative to the reference standard, wherein said concentration difference is selected from: an increase in the concentration of one or more urine metabolites selected from: TMAO and citrate; and
a decrease in the concentration of one or more urine metabolites selected from: creatinine, and lactate;
wherein said concentration differences correlate with the presence of MS.

2. The method of claim 1, wherein said metabolites comprise
(a) four or more blood metabolites selected from: fatty acid, N-acetyl species, glucose, phosphocholine, and L-glutamine; or
(b) five blood metabolites: fatty acid, N-acetyl species, glucose, phosphocholine, and L-glutamine
wherein said N-acetyl species is N-acetyl aspartate and/or N-acetyl glycoprotein; and/or wherein the fatty acid has a resonance value of δ0.90, br, C*H₃,* δ1.30, br, C*H₂*C*H₂*C*H₃,*δ1.55*,* br, C*H₂*C*H₃,* δ 2.05, br, C*H₂*C*H*=*,* δ 2.25, br, C*H₂*C*OOH,* δ5.35, br, C*H₂*C*H*= relative to a methyl resonance of 3-trimethylsilyl-1-propionate (TSP) defined at 0.00 ppm as determinable by ¹H-NMR.

3. The method of claim 1 or 2, wherein said metabolites comprise three or more blood metabolites selected from: glucose, phosphocholine, fatty acid, and lactate;
wherein the fatty acid has a resonance value of δ0.90, br, C*H₃,* δ1.30, br, C*H₂*C*H₂*C*H₃,*δ1.55*,* br, C*H₂*C*H₃,* δ 2.05, br, C*H₂*C*H*=*,* δ 2.25, br, C*H₂*C*OOH,* δ5.35, br, C*H₂*C*H*= relative to a methyl resonance of 3-trimethylsilyl-1-propionate (TSP) defined at 0.00 ppm as determinable by ¹H-NMR.

4. The method of claim 3, wherein said concentration differences are selected from:
a decrease in the concentration of one or more blood metabolites selected from: phosphocholine, lactate, N-acetyl species, wherein said N-acetyl species is N-acetyl aspartate and/or N-acetyl glycoprotein, and glucose; or
wherein said concentration differences are selected from:
a decrease in the concentration of one or more blood metabolites selected from: glucose, phosphocholine, fatty acid, wherein the fatty acid has a resonance value of δ0.90, br, C*H₃,* δ1.30, br, C*H₂*C*H₂*C*H₃,*δ1.55*,* br, C*H₂*C*H₃,* δ 2.05, br, C*H₂*C*H*=*,* δ 2.25, br, C*H₂*C*OOH,* δ5.35, br, C*H₂*C*H*= relative to a methyl resonance of 3-trimethylsilyl-1-propionate (TSP) defined at 0.00 ppm as determinable by ¹H-NMR, and lactate.

5. The method of claim 1 or 2, wherein said metabolites comprise
(a) three or more urine metabolites selected from: TMAO, citrate, creatinine, and lactate; or
(b) the following four urine metabolites: TMAO, citrate, creatinine, and lactate.

6. The method of claim 1 or 2, wherein said metabolites comprise:
(a) three or more blood metabolites selected from: fatty acid, lactate, alanine, phosphocholine, beta-hydroxybutyrate, N-acetyl species, and glucose; or
(b) four or more blood metabolites selected from: fatty acid, lactate, alanine, phosphocholine, beta-hydroxybutyrate, N-acetyl species, and glucose; or
(c) five or more blood metabolites selected from: fatty acid, lactate, alanine, phosphocholine, beta-hydroxybutyrate, N-acetyl species, and glucose; or
(d) six or more blood metabolites selected from: fatty acid, lactate, alanine, phosphocholine, beta-hydroxybutyrate, N-acetyl species, and glucose; or
(e) the following seven blood metabolites: fatty acid, lactate, alanine, phosphocholine, beta-hydroxybutyrate, N-acetyl species, and glucose;
wherein said N-acetyl species is N-acetyl aspartate and/or N-acetyl glycoprotein; and/or wherein the fatty acid has a resonance value of δ0.90, br, C*H₃,* δ1.30, br, C*H₂*C*H₂*C*H₃,*δ1.55*,* br, C*H₂*C*H₃,* δ 2.05, br, C*H₂*C*H*=, δ 2.25, br, C*H₂COOH*, δ5.35, br, C*H₂*C*H*= relative to a methyl resonance of 3-trimethylsilyl-1-propionate (TSP) defined at 0.00 ppm as determinable by ¹H-NMR,.

7. The method of claim 1, wherein said metabolites comprise
(a) three or more blood metabolites selected from: L-glutamine, alanine, glucose, phosphocholine, and lactate; or
(b) four or more blood metabolites selected from: L-glutamine, alanine, glucose, phosphocholine, and lactate; or
(c) the following five blood metabolites: L-glutamine, alanine, glucose, phosphocholine, and lactate.

8. The method of any preceding claim, wherein the concentrations of the metabolites are determined using a technique selected from: Nuclear Magnetic Resonance (NMR) spectroscopy, mass spectrometry, HPLC-UV, and infrared spectrometry.

## Patentansprüche

1. In-vitro-Verfahren zum Diagnostizieren von Multipler Sklerose (MS) in einer Testperson, das Folgendes beinhaltet:
a.
(i) Feststellen der relativen Konzentrationen von drei oder mehr Metaboliten in einer Probe von der genannten Person, wobei die genannten drei oder mehr Metabolite ausgewählt sind aus:
Blutmetaboliten, wobei die genannten Blutmetabolite Folgendes umfassen: Phosphocholin, Lactat, N-Acetylspezies, Fettsäure, Glucose und L-Glutamin;
(ii) Vergleichen der relativen Konzentrationen der genannten drei oder mehr Metabolite in der Probe mit den relativen Konzentrationen derselben Metabolite in wenigstens einem Referenzstandard von einer Person, die keine MS hat;
und
(iii) Identifizieren einer Konzentrationsdifferenz für jeden der genannten drei oder mehr Metabolite in der Probe relativ zum Referenzstandard, wobei die genannte Konzentrationsdifferenz ausgewählt ist aus: einer Zunahme der Konzentration von einem oder mehreren Blutmetaboliten, ausgewählt aus: Fettsäure, N-Acetylspezies und Lactat; und
einer Abnahme der Konzentration der Blutmetabolite Glucose und Phosphocholin; oder
wobei die genannte Konzentrationsdifferenz ausgewählt ist aus:
einer Zunahme der Konzentration von einem oder mehreren Blutmetaboliten, ausgewählt aus: Fettsäure, N-Acetylspezies und L-Glutamin;
und
eine Abnahme der Konzentration der Blutmetabolite Glucose und Phosphocholin;
wobei die genannten Konzentrationsdifferenzen mit der Anwesenheit von MS korrelieren;
wobei die genannte N-Acetylspezies N-Acetylaspartat und/oder N-Acetylglycoprotein sind;
und/oder
wobei die Fettsäure einen Resonanzwert von δ0,90, br, C*H₃,* δ1,30, br, C*H₂*C*H₂*C*H₃,* δ1,55*,* br, C*H₂CH₃,* δ 2,05, br, C*H₂*C*H*=*,* δ 2,25, br, C*H₂*C*OOH,* δ5,35, br, C*H₂*C*H*= relativ zu einer Methylresonanz von 3-Trimethylsilyl-1-propionat (TSP) hat, definiert bei 0,00 ppm, wie anhand von ¹H-NMR feststellbar;
und/oder
b.
(i) Feststellen der relativen Konzentrationen von zwei oder mehr Metaboliten in einer Probe von der genannten Person, wobei die genannten zwei oder mehr Metabolite ausgewählt sind aus: Urinmetaboliten, wobei die genannten Urinmetabolite Folgendes umfassen: Citrat, Creatinin, Lactat und Trimethylamin-N-oxid (TMAO);
(ii) Vergleichen der relativen Konzentrationen der genannten zwei oder mehr Metabolite in der Probe mit den relativen Konzentrationen derselben Metabolite in wenigstens einem Referenzstandard von einer Person, die keine MS hat;
und
(iii) Identifizieren einer Konzentrationsdifferenz für jeden der genannten zwei oder mehr Metabolite in der Probe relativ zu dem Referenzstandard, wobei die genannte Konzentrationsdifferenz ausgewählt ist aus: einer Zunahme der Konzentration von einem oder mehreren Urinmetaboliten, ausgewählt aus: TMAO und Citrat; und
einer Abnahme der Konzentration von einem oder mehreren Urinmetaboliten, ausgewählt aus:
Creatinin und Lactat;
wobei die genannten Konzentrationsdifferenzen mit der Anwesenheit von MS korrelieren.

2. Verfahren nach Anspruch 1, wobei die genannten Metabolite Folgendes umfassen:
(a) vier oder mehr Blutmetabolite, ausgewählt aus: Fettsäure, N-Acetylspezies, Glucose, Phosphocholin und L-Glutamin; oder
(b) fünf Blutmetabolite: Fettsäure, N-Acetylspezies, Glucose, Phosphocholin und L-Glutamin,
wobei die genannte N-Acetylspezies N-Acetylaspartat und/oder N-Acetylglycoprotein ist; und/oder
wobei die Fettsäure einen Resonanzwert von δ0,90, br, C*H₃,* δ1,30, br, C*H₂*C*H₂*C*H₃,* δ1*,*55*,* br, C*H₂CH₃,* δ 2,05, br, C*H₂*C*H*=*,* δ 2,25, br, C*H₂*C*OOH,* δ5,35, br, C*H₂*C*H*= relativ zu einer Methylresonanz von 3-Trimethylsilyl-1-propionat (TSP) hat, definiert bei 0,00 ppm, wie anhand von ¹H-NMR feststellbar.

3. Verfahren nach Anspruch 1 oder 2, wobei die genannten Metabolite drei oder mehr Blutmetabolite umfassen, ausgewählt aus: Glucose, Phosphocholin, Fettsäure und Lactat;
wobei die Fettsäure einen Resonanzwert von δ0,90, br, C*H₃,* δ1,30, br, C*H₂*C*H₂*C*H₃,* δ1,55*,* br, C*H₂CH₃,* δ 2,05, br, C*H₂*C*H*=*,* δ 2,25, br, C*H₂*C*OOH,* δ5,35, br, C*H₂*C*H*= relativ zu einer Methylresonanz von 3-Trimethylsilyl-1-propionat (TSP) hat, definiert bei 0,00 ppm, wie anhand von ¹H-NMR feststellbar.

4. Verfahren nach Anspruch 3, wobei die genannten Konzentrationsdifferenzen ausgewählt sind aus:
einer Abnahme der Konzentration von einem oder mehreren Blutmetaboliten, ausgewählt aus: Phosphocholin, Lactat, N-Acetylspezies, wobei die genannte N-Acetylspezies N-Acetylaspartat und/oder N-Acetylglycoprotein und Glucose ist; oder
wobei die genannten Konzentrationsdifferenzen ausgewählt sind aus:
einer Abnahme der Konzentration von einem oder mehreren Blutmetaboliten, ausgewählt aus: Glucose, Phosphocholin, Fettsäuren, wobei die Fettsäure einen Resonanzwert von δ0,90, br, C*H₃,* δ1,30, br, C*H₂*C*H₂*C*H₃,* δ1,55*,* br, C*H₂CH₃,* δ 2,05, br, C*H₂*C*H*=*,* δ 2,25, br, C*H₂*C*OOH,* δ5,35, br, C*H₂*C*H*= relativ zu einer Methylresonanz von 3-Trimethylsilyl-1-propionat (TSP) hat, definiert bei 0,00 ppm, wie durch ¹H-NMR feststellbar, und Lactat.

5. Verfahren nach Anspruch 1 oder 2, wobei die genannten Metabolite Folgendes umfassen:
(a) drei oder mehr Urinmetabolite, ausgewählt aus: TMAO, Citrat, Creatinin und Lactat; oder
(b) die folgenden vier Urinmetabolite: TMAO, Citrat, Creatinin und Lactat.

6. Verfahren nach Anspruch 1 oder 2, wobei die genannten Metabolite Folgendes umfassen:
(a) drei oder mehr Blutmetabolite ausgewählt aus: Fettsäure, Lactat, Alanin, Phosphocholin, beta-Hydroxybutyrat, N-Acetylspezies und Glucose; oder
(b) vier oder mehr Blutmetabolite ausgewählt aus: Fettsäure, Lactat, Alanin, Phosphocholin, beta-Hydroxybutyrat, N-Acetylspezies und Glucose; oder
(c) fünf oder mehr Blutmetabolite ausgewählt aus: Fettsäure, Lactat, Alanin, Phosphocholin, beta-Hydroxybutyrat, N-Acetylspezies und Glucose; oder
(d) sechs oder mehr Blutmetabolite ausgewählt aus: Fettsäure, Lactat, Alanin, Phosphocholin, beta-Hydroxybutyrat, N-Acetylspezies und Glucose; oder
(e) die folgenden sieben Blutmetabolite: Fettsäure, Lactat, Alanin, Phosphocholin, beta-Bydroxybutyrat, N-Acetylspezies und Glucose;
wobei die genannte N-Acetylspezies N-Acetylaspartat und/oder N-Acetylglycoprotein ist; und/oder
wobei die Fettsäure einen Resonanzwert von δ0,90, br, C*H₃,* δ1,30, br, C*H₂*C*H₂*C*H₃,* δ1*,*55*,* br, C*H₂CH₃,* δ 2,05, br, C*H₂*C*H*=*,* δ 2,25, br, C*H₂*C*OOH,* δ5,35, br, C*H₂*C*H*= relativ zu einer Methylresonanz von 3-Trimethylsilyl-1-propionat (TSP) hat, definiert bei 0,00 ppm, wie anhand von ¹H-NMR feststellbar.

7. Verfahren nach Anspruch 1, wobei die genannten Metabolite Folgendes umfassen:
(a) drei oder mehr Blutmetabolite, ausgewählt aus: L-Glutamin, Alanin, Glucose, Phosphocholin und Lactat; oder
(b) vier oder mehr Blutmetabolite ausgewählt aus: L-Glutamin, Alanin, Glucose, Phosphocholin und Lactat; oder
(c) die folgenden fünf Blutmetabolite: L-Glutamin, Alanin, Glucose, Phosphocholin und Lactat.

8. Verfahren nach einem vorherigen Anspruch, wobei die Konzentrationen der Metabolite mit einer Technik bestimmt werden, die ausgewählt ist aus: NMR-(Nuclear Magnetic Resonance)-Spektroskopie, Massenspektrometrie, HPLC-W und Infrarotspektrometrie.

## Revendications

1. Procédé *in vitro* de diagnostic de la sclérose en plaques (SP) chez un sujet expérimental humain, comprenant :
a.
(i) la détermination des concentrations relatives de trois métabolites ou plus dans un échantillon provenant dudit sujet, lesdits trois métabolites ou plus étant sélectionnés parmi :
des métabolites du sang, lesdits métabolites du sang comprenant : la phosphocholine, un lactate, une espèce N-acétyle, un acide gras, le glucose, et la L-glutamine ;
(ii) la comparaison des concentrations relatives desdits trois métabolites ou plus dans l'échantillon avec les concentrations relatives des mêmes métabolites dans au moins un étalon de référence provenant d'un sujet qui n'est pas atteint de la SP ;
et
(iii) l'identification d'une différence de concentration pour chacun desdits trois métabolites ou plus dans l'échantillon relativement à l'étalon de référence, ladite différence de concentration étant sélectionné parmi : une augmentation de la concentration d'un ou plusieurs métabolites du sang sélectionnés parmi : un acide gras, une espèce N-acétyle, et un lactate ; et
une diminution de la concentration des métabolites du sang glucose et phosphocholine ; ou
dans lequel ladite différence de concentration est sélectionnée parmi :
une augmentation de la concentration d'un ou plusieurs métabolites du sang sélectionnés parmi : un acide gras, une espèce N-acétyle, et la L-glutamine ;
et
une diminution de la concentration des métabolites du sang glucose et phosphocholine ;
dans lequel lesdites différences de concentration sont en corrélation avec la présence de la SP ;
dans lequel ladite espèce N-acétyle est l'aspartate de N-acétyle et/ou une glycoprotéine contenant un groupe N-acétyle ;
et/ou
dans lequel l'acide gras a une valeur de résonance de δ0,90, br, C*H₃,* δ1,30, br, C*H₂*C*H₂*C*H₃,* δ1,55, br, C*H₂CH₃,* δ2,05, br, C*H₂*C*H*=*,* δ2,25, br, C*H₂*C*OOH,* δ5,35, br, C*H₂*C*H*= relativement à une résonance des protons méthyliques du 3-triméthylsilyl-1-propionate (TSP) définie à 0,00 ppm comme déterminable par RMN ¹H ;
et/ou
b.
(i) la détermination des concentrations relatives de deux métabolites ou plus dans un échantillon provenant dudit sujet, lesdits deux métabolites ou plus étant sélectionnés parmi : des métabolites de l'urine, lesdits métabolites de l'urine comprenant : un citrate, la créatinine, un lactate et le N-oxyde de triméthylamine (TMAO) ;
(ii) la comparaison des concentrations relatives desdits deux métabolites ou plus dans l'échantillon avec les concentrations relatives des mêmes métabolites dans au moins un étalon de référence provenant d'un sujet qui n'est pas atteint de la SP ;
et
(iii) l'identification d'une différence de concentration pour chacun desdits deux métabolites ou plus dans l'échantillon relativement à l'étalon de référence, ladite différence de concentration étant sélectionné parmi : une augmentation de la concentration d'un ou plusieurs métabolites de l'urine sélectionnés parmi : le TMAO et un citrate ; et
une diminution de la concentration d'un ou plusieurs métabolites de l'urine sélectionnés parmi : la créatinine, et un lactate ;
dans lequel lesdites différences de concentration sont en corrélation avec la présence de la SP.

2. Procédé selon la revendication 1, dans lequel lesdits métabolites comprennent :
(a) quatre métabolites du sang ou plus sélectionnés parmi : un acide gras, une espèce N-acétyle, le glucose, la phosphocholine, et la L-glutamine ; ou
(b) cinq métabolites du sang parmi : un acide gras, une espèce N-acétyle, le glucose, la phosphocholine, et la L-glutamine ;
dans lequel ladite espèce N-acétyle est l'aspartate de N-acétyle et/ou une glycoprotéine contenant un groupe N-acétyle ; et/ou
dans lequel l'acide gras a une valeur de résonance de δ0,90, br, C*H₃,* δ1,30, br, C*H₂*C*H₂*C*H₃,* δ1,55, br, C*H₂CH₃,* δ2,05, br, C*H₂*C*H*=*,* δ2,25, br, C*H₂*C*OOH,* δ5,35, br, C*H₂*C*H*= relativement à une résonance des protons méthyliques du 3-triméthylsilyl-1-propionate (TSP) définie à 0,00 ppm comme déterminable par RMN ¹H.

3. Procédé selon la revendication 1 ou 2, dans lequel lesdits métabolites comprennent trois métabolites du sang ou plus sélectionnés parmi : le glucose, la phosphocholine, un acide gras, et un lactate ;
dans lequel l'acide gras a une valeur de résonance de δ0,90, br, C*H₃,* δ1,30, br, C*H₂*C*H₂*C*H₃,* δ1,55, br, C*H₂CH₃,* δ2,05, br, C*H₂*C*H*=*,* δ2,25, br, C*H₂*C*OOH,* δ5,35, br, C*H₂*C*H*= relativement à une résonance des protons méthyliques du 3-triméthylsilyl-1-propionate (TSP) définie à 0,00 ppm comme déterminable par RMN ¹H.

4. Procédé selon la revendication 3, dans lequel lesdites différences de concentration sont sélectionnées parmi :
une diminution de la concentration d'un ou plusieurs métabolites du sang sélectionnés parmi :
la phosphocholine, un lactate, une espèce N-acétyle, ladite espèce N-acétyle étant l'aspartate de N-acétyle et/ou une glycoprotéine contenant un groupe N-acétyle, et le glucose ; ou
dans lequel lesdites différences de concentration sont sélectionnées parmi :
une diminution de la concentration d'un ou plusieurs métabolites du sang sélectionnés parmi : le glucose, la phosphocholine, un acide gras, l'acide gras ayant une valeur de résonance de δ0,90, br, C*H₃,* δ1,30, br, C*H₂*C*H₂*C*H₃,* δ1,55, br, C*H₂*C*H₃,* δ2,05, br, C*H₂*C*H*=*,* δ2,25, br, C*H₂*C*OOH,* δ5,35, br, C*H₂*C*H*= relativement à une résonance des protons méthyliques du 3-triméthylsilyl-1-propionate (TSP) définie à 0,00 ppm comme déterminable par RMN ¹H, et un lactate.

5. Procédé selon la revendication 1 ou 2, dans lequel lesdits métabolites comprennent :
(a) trois métabolites de l'urine ou plus sélectionnés parmi : le TMAO, un citrate, la créatinine, et un lactate ; ou
(b) les quatre métabolites de l'urine suivants : le TMAO, un citrate, la créatinine, et un lactate.

6. Procédé selon la revendication 1 ou 2, dans lequel lesdits métabolites comprennent :
(a) trois métabolites du sang ou plus sélectionnés parmi : un acide gras, un lactate, une alanine, la phosphocholine, le bêta-hydroxybutyrate, une espèce N-acétyle, et le glucose ; ou
(b) quatre métabolites du sang ou plus sélectionnés parmi : un acide gras, un lactate, une alanine, la phosphocholine, le bêta-hydroxybutyrate, une espèce N-acétyle, et le glucose ; ou
(c) cinq métabolites du sang ou plus sélectionnés parmi : un acide gras, un lactate, une alanine, la phosphocholine, le bêta-hydroxybutyrate, une espèce N-acétyle, et le glucose ; ou
(d) six métabolites du sang ou plus sélectionnés parmi : un acide gras, un lactate, une alanine, la phosphocholine, le bêta-hydroxybutyrate, une espèce N-acétyle, et le glucose ; ou
(e) les sept métabolites du sang suivants : un acide gras, un lactate, une alanine, la phosphocholine, le bêta-hydroxybutyrate, une espèce N-acétyle, et le glucose ;
dans lequel ladite espèce N-acétyle est l'aspartate de N-acétyle et/ou une glycoprotéine contenant un groupe N-acétyle ; et/ou
dans lequel l'acide gras a une valeur de résonance de δ0,90, br, C*H₃,* δ1,30, br, C*H₂*C*H₂*C*H₃,* δ1,55, br, C*H₂CH₃,* δ2,05, br, C*H₂*C*H*=*,* δ2,25, br, C*H₂*C*OOH,* δ5,35, br, C*H₂*C*H*= relativement à une résonance des protons méthyliques du 3-triméthylsilyl-1-propionate (TSP) définie à 0,00 ppm comme déterminable par RMN ¹H.

7. Procédé selon la revendication 1, dans lequel lesdits métabolites comprennent :
(a) trois métabolites du sang ou plus sélectionnés parmi : la L-glutamine, une alanine, le glucose, la phosphocholine, et un lactate ; ou
(b) quatre métabolites du sang ou plus sélectionnés parmi : la L-glutamine, une alanine, le glucose, la phosphocholine, et un lactate ; ou
(c) les cinq métabolites du sang suivants : la L-glutamine, une alanine, le glucose, la phosphocholine, et un lactate.

8. Procédé selon l'une quelconque des revendications précédentes, dans lequel les concentrations des métabolites sont déterminées en utilisant une technique sélectionnée parmi : la spectroscopie de résonance magnétique nucléaire (RMN), la spectrométrie de masse, la CLHP-UV, et la spectrométrie infrarouge.
